# EUROPEAN PATENT SPECIFICATION

(11) **EP 3 936 850 B1**
(45) Date of publication and mention of the grant of the patent: **09.08.2023**
(21) Application number: 21179036.5
(22) Date of filing: 22.12.2016
(51) Int. Cl.: G01N 1/00, G01N 33/50, G01N 33/68, G01N 33/58, G01N 33/543

(54) **A METHOD TO IDENTIFY AN APPROACH FOR ACHIEVING MAMMALIAN FERTILIZATION AND TIME PERIOD FOR INSEMINATION**
VERFAHREN ZUR IDENTIFIZIERUNG EINES ANSATZES ZUR ERZIELUNG VON SÄUGETIERBEFRUCHTUNG UND ZEITRAUM ZUR BESAMUNG
PROCÉDÉ D'IDENTIFICATION D'UNE APPROCHE PERMETTANT DE RÉALISER UNE FÉCONDATION CHEZ LES MAMMIFÈRES ET D'IDENTIFICATION DE LA PÉRIODE D'INSÉMINATION

(30) Priority: 23.12.2015 US 201562387348 P; 15.01.2016 US 201662279315 P; 28.04.2016 US 201662328876 P; 28.04.2016 US 201662328885 P; 15.11.2016 US 201662422279 P
(43) Date of publication of application: 12.01.2022
(62) Divisional of application: 16880078.7
(73) Proprietor: Androvia Life Sciences LLC, Mountainside, NJ 07092 (US)
(72) Inventor: TRAVIS, Alexander, J., Ithaca, NY 14850 (US); CARDONA, Cristina, Highland Park, NJ 08904 (US); MOODY, Melissa, A., Brick, NJ 08724 (US); SIMPSON, Alana, J., Summit,NJ 07901 (US); OSTERMEIER, G. Charles, Gillette, NJ 07933 (US)
(74) Representative: Gill Jennings & Every LLP

(56) References cited:
- WO-A2-2005/009222
- US-A- 5 994 086
- Theodore Paniza ET AL: "A Bioassay to Measure Fertilization Competence of Human Spermatozoa Results Potential Clinical Application", , 2014, XP055598765, Retrieved from the Internet: URL:http://icsicenter.squarespace.com/s/Pa niza_ASRM_10_14_2014.pdf [retrieved on 2019-06-24]
- Aj Travis: "CAPACITATION DEFECTS ARE COMMON IN MEN QUESTIONING THEIR FERTILITYAND ARE INDEPENDENT OF STANDARD SEMEN ANALYSIS PARAMETERS.", , 18 October 2016 (2016-10-18), XP055618810, Retrieved from the Internet: URL:https://www.fertstert.org/article/S001 5-0282(16)62102-7/pdf [retrieved on 2019-09-05]
- MELISSA A. MOODY ET AL: "Validation of a laboratory-developed test of human sperm capacitation", MOLECULAR REPRODUCTION AND DEVELOPMENT, vol. 84, no. 5, 18 April 2017 (2017-04-18) , pages 408-422, XP055437335, NEW YORK, NY, US ISSN: 1040-452X, DOI: 10.1002/mrd.22801

## Description

### FIELD OF THE INVENTION

This invention relates generally to the field of male fertility and more specifically provides methods of identifying a reproductive approach to use in order to achieve fertilization and/or modifying the time period at which insemination is performed in order to achieve fertilization.

### BACKGROUND

The diagnosis of male infertility is based predominantly on the results of standard semen analysis for concentration, total motility, progressive motility, volume, pH, viscosity and/or morphology. However, measurements of sperm morphology, motility and concentration do not assess fertilizing potential, including the complex changes that sperm undergo during residence within the female reproductive tract. In addition to the challenges associated with assessing fertilizing potential, cryopreservation is often used to preserve sperm cells and preserve male fertility for extended periods of time. Unfortunately, freezing and thawing can negatively affect sperm viability and function. Cryopreservation is reported to alter capacitation and shift/limit the fertilization window.

Newly developed fertility tests should determine the ability of sperm to fertilize, as well as initiate and maintain pregnancy (Oehninger et al., "Sperm functional tests," Fertil Steril. 102: 1528-33 (2014); Wang et al., "Limitations of semen analysis as a test of male fertility and anticipated needs from newer tests," Fertil Steril. 102: 1502-07 (2014)). While freshly ejaculated spermatozoa appear morphologically mature and motile, they are fertilization incompetent. They must first undergo a maturational process known as "capacitation," which renders them capable of fertilization (Austin, "The capacitation of the mammalian sperm," Nature, 170: 326 (1952); Chang, "Fertilizing capacity of spermatozoa deposited into the fallopian tubes," Nature, 168: 697-8 (1951)). In most species, capacitation is dependent upon the removal of sterols from the sperm plasma membrane (sterol efflux) and the influx of bicarbonate and calcium ions (Baldi et al., "Intracellular calcium accumulation and responsiveness to progesterone in capacitating human spermatozoa," J Androl. 12: 323-30 (1991); Bedu-Addo et al., "Bicarbonate and bovine serum albumin reversibly 'switch' capacitation-induced events in human spermatozoa," Mol Hum Reprod., 11: 683-91 (2005); Cohen et al., "Lipid modulation of calcium flux through Cav2.3 regulates acrosome exocytosis and fertilization," Dev Cell. 28: 310-21 (2014); Gadella et al., "Bicarbonate and its role in mammalian sperm function," Anim. Reprod. Sci. 82: 307-19 (2004)). The efflux of sterols that occurs during sperm capacitation changes membrane fluidity patterns and allows for the redistribution of specific membrane components (Cohen et al. 2014; Cross, "Role of cholesterol in sperm capacitation," Biol Reprod. 59: 7-11 (1998); Selvaraj et al., "Mechanisms underlying the micron-scale segregation of sterols and GM1 in live mammalian sperm," J Cell Physiol. 218: 522-36 (2007); Selvaraj et al, "GM1 dynamics as a marker for membrane changes associated with the process of capacitation in murine and bovine spermatozoa," J Androl. 28: 588-99 (2009))

Currently, there are few if any sensitive and simple capacitation biomarkers suitable for clinical application. For example, the phosphorylation of tyrosine residues has been well detailed in association with capacitation (Battistone et al., "Functional human sperm capacitation requires both bicarbonate-dependent PKA activation and down-regulation of Ser/Thr phosphatases by Src family kinases," Mol, Human Reprod. 19: 570-80 (2013); Osheroff et al., "Regulation of human sperm capacitation by a cholesterol efflux-stimulated signal transduction pathway leading to protein kinase A-mediated up-regulation of protein tyrosine phosphorylation," Mol, Human Reprod. 5: 1017-26 (1999); Visconti et al.. "Capacitation of mouse spermatozoa. I. Correlation between the capacitation state and protein tyrosine phosphorylation," Development, 121: 1129-37 (1995)). However, evaluating these events can take multiple days and provide only a semi-quantitative assessment, making it inappropriate for the clinical evaluation of male fertility.

It has been known for some time that sperm must undergo sterol efflux to become fertilization competent (Osheroff et al. 1999; Travis et al., "The role of cholesterol efflux in regulating the fertilization potential of mammalian spermatozoa," The Journal of Clinical Investigation, 110: 731-36 (2002)). In addition, cholesterol and other lipids, such as the ganglioside G_{M1}, are organized into microdomains within the sperm's plasma membrane (Asano et al., "Biochemical characterization of membrane fractions in murine sperm: Identification of three distinct sub-types of membrane rafts," J Cell Physiol., 218: 537-48 (2009); Asano et al., "Characterization of the proteomes associating with three distinct membrane raft sub-types in murine sperm," Proteomics, 10: 3494-505 (2010); Travis et al., "Expression and localization of caveolin-1, and the presence of membrane rafts, in mouse and Guinea pig spermatozoa," Dev Biol., 240: 599-610 (2001); Selvaraj et al. 2009). These membrane rafts consolidate signaling pathways, making them sensible candidates for mediating sperm function. Interestingly, predictable changes in G_{M1} localization patterns have been measured both in mouse and bull sperm that have been stimulated for capacitation (Selvaraj et al. 2007). What's more, G_{M1} regulates the activity of an R-type calcium channel, triggering a transient calcium flux that is essential for acrosome exocytosis and thus successful fertilization (Cohen et al. 2014). These findings substantiate the use of G_{M1} localization patterns to assess sperm function and accordingly male fertility.

Various G_{M1} localization patterns have been identified and associated with capacitation or non-capacitation. In particular, apical acrosome (AA) G_{M1} localization patterns and acrosomal plasma membrane (APM) G_{M1} localization patterns have been associated with capacitation in bovine and human sperm. Sperm capacitation can be quantitatively expressed as a Cap-Score^{™} value, generated via the Cap-Score^{™} Sperm Function Test ("Cap-Score^{™} Test" or "Cap-Score"), is defined as ([number of apical acrosome (AA) G_{M1} localization patterns + number of acrosomal plasma membrane (APM) G_{M1} localization patterns]/total number of G_{M1} labeled localization patterns) where the number of each localization pattern is measured and then ultimately converted to a percentage score. In addition to APM G_{M1} localization patterns and AA G_{M1} localization patterns, the other labeled localization patterns included Lined-Cell G_{M1} localization patterns, intermediate (INTER) G_{M1} localization patterns, post acrosomal plasma membrane (PAPM) G_{M1} localization patterns, apical acrosome/post acrosome (AA/PA) G_{M1} localization patterns, equatorial segment (ES) G_{M1} localization patterns, and diffuse (DIFF) G_{M1} localization patterns. (Travis et al., "Impacts of common semen handling methods on sperm function," The Journal of Urology, 195 (4), e909 (2016)). Paniza et al presented a poster entitled "A bioassay to measure fertilization competence of human spermatozoa" at the American Society for Reproductive Medicine (ASRM) conference in October 2014, considering the correlation between G_{M1} localization assay scores and IUI results / natural conceptions.

### SUMMARY OF INVENTION

The present invention provides a method of using a difference in percentage of t₀-[AA G_{M1} localization patterns plus APM G_{M1} localization patterns] and percentage of ti-[AA G_{M1} localization patterns plus APM G_{M1} localization patterns] as an indicator of a time period for insemination and a reproductive approach for achieving mammalian fertilization comprising the steps of: (a) treating a first sample of t₀*-in vitro* capacitated sperm cells with a fixative and a fluorescence label; (b) treating a second sample of t₁*-in vitro* capacitated sperm cells with a fixative and a fluorescence label, wherein the capacitated sperm cells of the first sample and the second sample are treated *in vitro* with capacitation conditions for a capacitation time period ranging between 0.5 hours to 3 hours; 3 hours to 12 hours; 6 hours to 12 hours; 3 hours to 24 hours; 12 hours to 24 hours; or 18 hours to 24 hours; (c) obtaining one or more t₀-fluorescence images displaying one or more G_{M1} localization patterns associated with t₀-fluorescence labeled *in vitro* capacitated sperm cells, (d) obtaining one or more t₁-fluorescence images displaying one or more G_{M1} localization patterns associated with t₁-fluorescence labeled *in vitro* capacitated sperm cells, said t₀-fluorescenceimages and t₁-fluorescence images being obtained at post *in vitro* capacitation times, wherein t₁ is greater than t₀; (e) measuring a number of apical acrosome (AA) G_{M1} localization patterns, a number of acrosomal plasma membrane (APM) G_{M1} localization patterns and a total number of G_{M1} localization patterns for the t₀-fluorescence labeled *in vitro* capacitated sperm cells displayed in the t₀-fluorescenceimages to determine the percentage of t₀-[AA G_{M1} localization patterns plus APM G_{M1} localization patterns]; (f) measuring a number of apical acrosome (AA) G_{M1} localization patterns, a number of acrosomal plasma membrane (APM) G_{M1} localization patterns and a total number of G_{M1} localization patterns for the t₁-fluorescence labeled *in vitro* capacitated sperm cells displayed in the ti-fluorescence images to determine the percentage of ti-[AA G_{M1} localization patterns plus APM G_{M1} localization patterns]; (g) comparing the percentage of ti-[AA G_{M1} localization patterns plus APM G_{M1} localization patterns] to the percentage of t₀-[AA G_{M1} localization patterns plus APM G_{M1} localization patterns], wherein the comparison indicates an *in vivo* capacitation time selected from a late *in vivo* capacitation time greater than 12 hours or a standard *in vivo* capacitation time of 12 hours or less, (i) wherein a difference in percentage of t₀-[AA G_{M1} localization patterns plus APM G_{M1} localization patterns] and the percentage of ti-[AA G_{M1} localization patterns plus APM G_{M1} localization patterns] corresponding to: greater than one standard deviation from a standard of 35 % indicates a late *in vivo* capacitation time greater than 12 hours; or less than one standard deviation from a standard of 35 % indicates a standard *in vivo* capacitation time less than 12 hours; (i) wherein a reference percentage of [AA G_{M1} localization patterns plus APM G_{M1} localization patterns] corresponding to: greater than 35 % indicates a high fertility status; one standard deviation below 35 % indicates a medium fertility status; and two or more standard deviations below 35 % indicates a low fertility status; (iii) further wherein a difference in percentage of t₀-[AA G_{M1} localization patterns plus APM G_{M1} localization patterns] and the percentage of ti-[AA G_{M1} localization patterns plus APM G_{M1} localization patterns] corresponding to: greater than one standard deviation from a standard of 35 %, then a ti-fertility status is indicated based on a comparison of the percentage of measured ti-[AA G_{M1} localization patterns plus APM G_{M1} localization patterns] to the reference percentage of [AA G_{M1} localization patterns plus APM G_{M1} localization patterns]; or less than one standard deviation from a standard of 35 % then a ti-fertility status is indicated based on a comparison of the reference percentage of [AA G_{M1} localization patterns plus APM G_{M1} localization patterns] to the percentage of measured ti-[AA G_{M1} localization patterns plus APM G_{M1} localization patterns] or the percentage of measured t₀-[AA G_{M1} localization patterns plus APM G_{M1} localization patterns]; thereby indicating the time period for insemination and the reproductive approach to use in order to achieve fertilization based on the male's ti-fertility status and the *in vivo* capacitation time.

In an embodiment, if the male has at least normal sperm concentration and late *in vivo* capacitation time then (a) the reproductive approach for high ti-fertility status is selected from the group consisting of: modifying the timing of intercourse to late *in vivo* capacitation time; modifying the timing of intracervical insemination (ICI) to late *in vivo* capacitation time; modifying the timing of intrauterine insemination (IUI) to late *in vivo* capacitation time; pre-capacitating sperm prior to intracervical insemination, or pre-capacitating sperm prior to intrauterine insemination; (b) the reproductive approach for medium ti-fertility status is selected from group consisting of: modifying the timing of intracervical insemination (ICI) to late *in vivo* capacitation time; modifying the timing of intrauterine insemination (IUI) to late *in vivo* capacitation time; modifying the timing of *in vitro* fertilization (IVF) to late *in vivo* capacitation time; pre-capacitating sperm prior to intracervical insemination; intrauterine insemination (IUI); pre-capacitating sperm prior to intrauterine insemination; or pre-capacitating sperm prior to *in vitro* fertilization; and, (c) the reproductive approach for low ti-fertility status is selected from group consisting of: modifying the timing of *in vitro* fertilization (IVF) to late *in vivo* capacitation time; modifying the timing of intracytoplasmic sperm injection (ICSI) to late *in vivo* capacitation time; modifying the timing of gamete intra-fallopian transfer (GIFT) to late *in vivo* capacitation time; modifying the timing of subzonal insemination (SUZI) to late *in vivo* capacitation time; pre-capacitating sperm prior to *in vitro* fertilization, pre-capacitating sperm prior to intracytoplasmic sperm injection, pre-capacitating sperm prior to gamete intra-fallopian transfer, or pre-capacitating sperm prior to subzonal insemination.

In an embodiment, if the male has a less than normal sperm concentration and late *in vivo* capacitation time then, (a) the reproductive approach for ti-high fertility status is selected from the group consisting of: modifying the timing of intercourse to late *in vivo* capacitation time; modifying the timing of intracervical insemination (ICI) to late *in vivo* capacitation time; modifying the timing of intrauterine insemination (IUI) to late *in vivo* capacitation time; pre-capacitating sperm prior to intracervical insemination (ICI), or pre-capacitating sperm prior to intrauterine insemination (IUI); (b) the reproductive approach for ti-medium fertility status is selected from group consisting of: modifying the timing of intracervical insemination (ICI) to late *in vivo* capacitation time; modifying the timing of intrauterine insemination (IUI) to late *in vivo* capacitation time; modifying the timing of *in vitro* fertilization (IVF) to late *in vivo* capacitation time; pre-capacitating sperm prior to intracervical insemination, pre-capacitating sperm prior to intrauterine insemination; or pre-capacitating sperm prior to *in vitro* fertilization; and (c) the reproductive approach for ti-low fertility status is selected from group consisting of: modifying the timing of *in vitro* fertilization (IVF) to late *in vivo* capacitation time; intracytoplasmic sperm injection (ICSI), modifying the timing of intracytoplasmic sperm injection (ICSI) to late *in vivo* capacitation time; modifying the timing of gamete intra-fallopian transfer (GIFT) to late *in vivo* capacitation time; modifying the timing of subzonal insemination (SUZI) to late *in vivo* capacitation time, pre-capacitating sperm prior to *in vitro* fertilization, pre-capacitating sperm prior to intracytoplasmic sperm injection, pre-capacitating sperm prior to gamete intra-fallopian transfer, or pre-capacitating sperm prior to subzonal insemination.

In an embodiment, if the male has at least normal sperm concentration and standard *in vivo* capacitation time, then (a) the reproductive approach for high ti-fertility status is selected from the group consisting of: intercourse, intracervical insemination (ICI), pre-capacitating sperm prior to intracervical insemination, intrauterine insemination (IUI), or pre-capacitating sperm prior to intrauterine insemination; (b) the reproductive approach for medium ti-fertility status is selected from the group consisting of: intracervical insemination (ICI), pre-capacitating sperm prior to intracervical insemination; intrauterine insemination (IUI); pre-capacitating sperm prior to intrauterine insemination; or *in vitro* fertilization (IVF) or pre-capacitating sperm prior to *in vitro* fertilization; and (c) the reproductive approach for low ti-fertility status is selected from group consisting of: *in vitro* fertilization (IVF), pre-capacitating sperm prior to *in vitro* fertilization, intracytoplasmic sperm injection (ICSI), pre-capacitating sperm prior to intracytoplasmic sperm injection, gamete intra-fallopian transfer (GIFT), pre-capacitating sperm prior to gamete intra-fallopian transfer, subzonal insemination (SUZI), or pre-capacitating sperm prior to subzonal insemination.

In an embodiment, if the male has a less than normal sperm concentration and standard *in vivo* capacitation time, then (a) the reproductive approach for high ti-fertility status is selected from group consisting of: intracervical insemination (ICI), pre-capacitating sperm prior to intracervical insemination, intrauterine insemination (IUI) or pre-capacitating sperm prior to intrauterine insemination; (b) the reproductive approach for medium ti-fertility status is selected from group consisting of: intracervical insemination (ICI), pre-capacitating sperm prior to intracervical insemination, intrauterine insemination (IUI) or pre-capacitating sperm prior to intrauterine insemination; *in vitro* fertilization (IVF), pre-capacitating sperm prior to *in vitro* fertilization; and (c) the reproductive approach for low ti-fertility status is selected from group consisting of: *in vitro* fertilization (IVF), pre-capacitating sperm prior to *in vitro* fertilization, intracytoplasmic sperm injection (ICSI), pre-capacitating sperm prior to intracytoplasmic sperm injection, gamete intra-fallopian transfer (GIFT), pre-capacitating sperm prior to gamete intra-fallopian transfer, subzonal insemination (SUZI) or pre-capacitating sperm prior to subzonal insemination.

In an embodiment, when the reproductive approach corresponds to: (i) pre-capacitating sperm prior to *in vitro* fertilization, (ii) intracytoplasmic sperm injection (ICSI), (iii) gamete intra-fallopian transfer (GIFT), or (iv) subzonal insemination (SUZI), the time period for pre-capacitation prior to insemination corresponds to incubating sperm in media containing one or more stimuli for capacitation, for periods of 24 hours before insemination; 18 hours before insemination; 12 hours before insemination; 6 hours before insemination; 4 hours before insemination; 3 hours before insemination; or 1 hour before insemination. It is envisaged that the time period and/or the reproductive approach may also be based on one or more of the following: patient demographics, reproductive status of female partner, sperm concentration, total motility, progressive motility, semen volume, semen pH, semen viscosity and/or sperm morphology and combinations thereof. Further, it is envisaged that the one or more G_{M1} localization patterns may include AA G_{M1} localization pattern, APM G_{M1} localization pattern, Lined-Cell G_{M1} localization pattern, INTER G_{M1} localization pattern, PAPM G_{M1} localization pattern, AA/PA G_{M1} localization pattern, ES G_{M1} localization pattern, and DIFF G_{M1} localization pattern. The sperm cells may have been treated to cryopreservation procedures and stored prior to being treated *in vitro* with capacitation conditions.

### BRIEF DESCRIPTION OF THE DRAWINGS

The foregoing summary, as well as the following detailed description of the invention, will be better understood when read in conjunction with the appended drawings.

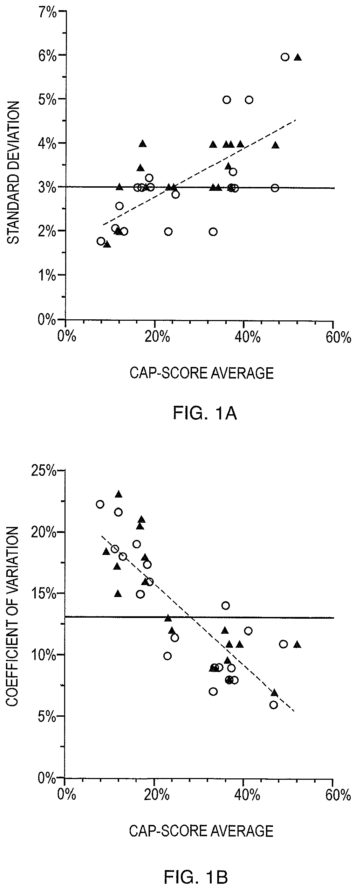

FIG. 1A illustrates a plot of the average Cap-Score is shown on the x-axis and the corresponding Standard Deviation is shown on the y-axis. The average SD for all images was found to be three (3) and is shown by the solid horizontal line. The dotted lines show the linear dependence of the SD (y = 0.06x + 0.02; r= 0.69; p=0.00).
FIG. 1B illustrates a plot of the average Cap-Score shown on the x-axis and Coefficient of Variation is shown on the y-axes. The CoV for all images was found to be thirteen (13) and is shown by the solid horizontal line. The dotted lines show the linear dependence of the CoV (y = -0.32x + 0.22; r=-0.84; p=0.00) to the Cap-Score average.

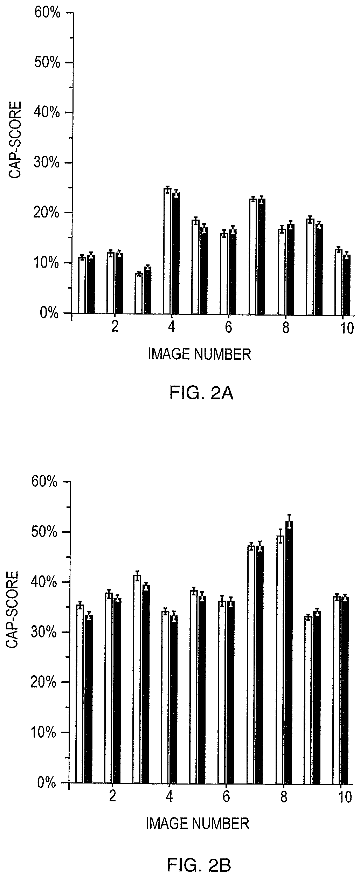

FIG. 2A illustrates the reproducibility of mean Cap-Scores between operators. Ten stitched images, containing up to 5,000 sperm each, were obtained for "less than normal" (more than one (1) SD below the mean Cap-Score result for a population of normal men. Two different readers determined Cap-Scores by randomly resampling each image 20 times and counting 150 cells each time (reader 1 open bars, reader 2 grey bars).
FIG. 2B illustrates the reproducibility of mean Cap-Scores between operators. Ten stitched images, containing up to 5,000 sperm each, were obtained for "presumed normal" classified as above one (1) SD below the mean Cap-Score result for a population of normal men. Two different readers determined Cap-Scores by randomly resampling each image 20 times and counting 150 cells each time (reader 1 open bars, reader 2 grey bars).

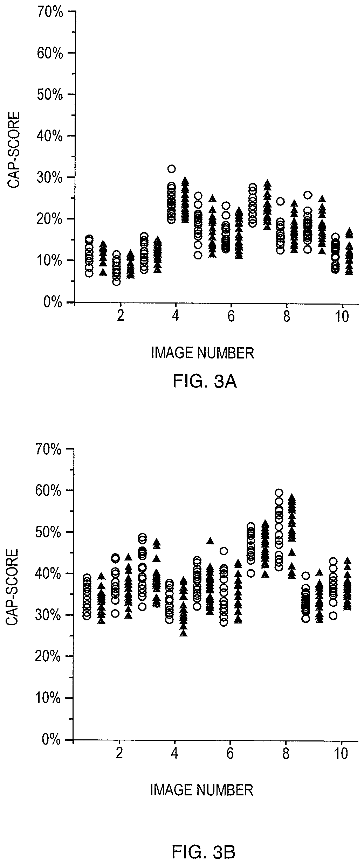

FIG. 3A illustrates the repeatability of Cap-Score variances between operators. Ten stitched images, containing up to 5,000 sperm each, were obtained for "less than normal" (more than one (1) SD below the mean Cap-Score result for a population of normal men. Two different readers determined Cap-Scores by randomly resampling each image 20 times and counting 150 cells each time (reader 1 open bars, reader 2 grey bars).
FIG. 3B illustrates the repeatability of Cap-Score variances between operators. Ten stitched images, containing up to 5,000 sperm each, were obtained for "presumed normal" classified as above one (1) SD below the mean Cap-Score result for a population of normal men. Two different readers determined Cap-Scores by randomly resampling each image 20 times and counting 150 cells each time (reader 1 open bars, reader 2 grey bars).

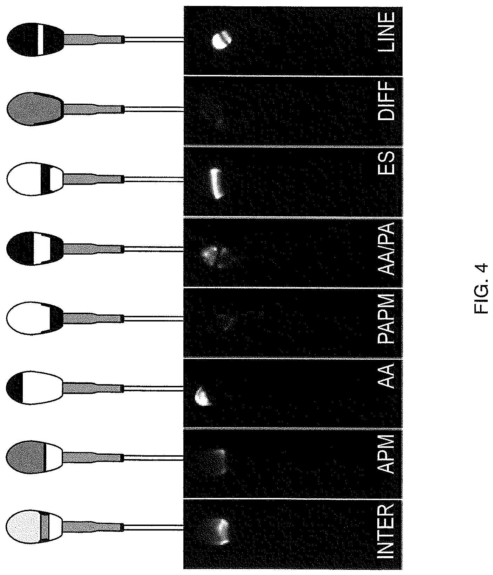

FIG. 4 illustrates various localization patterns of G_{M1} in normal human sperm and sperm from infertile males which form under in vitro capacitating conditions.

### DETAILED DESCRIPTION

As described herein, the time period for sperm capacitation among and within different males varies. It has been discovered that determining the time period for a male's sperm capacitation can be used to identify a time period for insemination and a reproductive approach to use during the insemination time period in order to achieve fertilization.

G_{M1} refers to monosialotetrahexosylganglioside and is a member of the ganglio series of gangliosides.

For human sperm, eight different G_{M1} localization patterns have been reported when the sperm was under in vitro capacitating conditions as illustrated in FIG. 4. To visualize the location patterns, the capacitated sperm were treated with labeling molecule for G_{M1}, such as cholera toxin b, which has a florescence detectable label on it.

INTER is characterized by a sperm cell where the vast majority of the fluorescence signal is in a band around the equatorial segment, with some signal in the plasma membrane overlying the acrosome. There is usually a gradient of the fluorescence signal, with the most at the equatorial segment and then progressively less toward the tip. There is often an increase in fluorescence signal intensity on the edges of the sperm head in the band across the equatorial segment.

Apical Acrosome "AA" is characterized by a sperm cell where the fluorescence signal is concentrated toward the apical tip, increased in brightness and reduced in area with signal.

Acrosomal Plasma Membrane "APM" is characterized by a sperm cell exhibiting a distributed fluorescence signal in the plasma membrane overlying the acrosome. APM signal is seen either from the bright equatorial INTER band moving apically toward the tip, or it can start further up toward the tip and be found in a smaller region, as it is a continuum with the AA.

Post-Acrosomal Plasma Membrane "PAPM" is characterized by a sperm cell where the fluorescence signal is exclusively in the post-acrosomal plasma membrane.

Apical Acrosome Post-Acrosome "AA/PA" is characterized by a sperm cell where the fluorescence signal is located both in the plasma membrane overlying the acrosome and post-acrosomal plasma membrane. The equatorial segment does not exhibit a fluorescence signal.

Equatorial Segment "ES" is characterized by a sperm cell having a bright fluorescence signal located solely in the equatorial segment. It may be accompanied by thickening of the sperm head across the equatorial region.

Diffuse "DIFF" is characterized by a sperm cell having a diffuse fluorescence signal located over the whole sperm head.

Lined-Cell is characterized by a sperm cell having a diffuse fluorescence signal ontop of the post-acrosomal region and at the plasma membrane overlying the acrosome as well as the bottom of the equatorial segment (i.e., the post acrosome/equatorial band). A fluorescence signal is missing around the equatorial segment.

The various G_{M1} localization patterns are identified by treating sperm cells with labeling molecule for G_{M1}, such as cholera toxin b, which has a florescence detectable label on it. The labeled sperm cells are then visualized using a fluorescence microscope as known to those of skill in the art.

Cap-Score is defined as the ratio of [the number of apical acrosome (AA) G_{M1} localization patterns + the number of acrosomal plasma membrane (APM) G_{M1} localization patterns] divided by [the total number of G_{M1} labeled localization patterns.] (Travis et al., "Impacts of common semen handling methods on sperm function," The Journal of Urology, 195 (4), e909 (2016)). To arrive at the number of different G_{M1} localization patterns, the number of, localization patterns, are counted for at least 100 sperm cells.

For the purposes of this application "t₀" corresponds to the number of hours after treating sperm cells with *in vitro* capacitation conditions and is selected from 0.1 hour to 5 hours; 0.1 hour to 8 hours; 0.1 to 12 hours; or 0.1 hour 18 hours.

For the purposes of this application "t₁" corresponds to the number of hours after treating sperm cells with *in vitro* capacitation conditions and is greater than 18 hours or greater than t₀.

For the purposes of this application images is understood to mean (i) digital images; (ii) G_{M1} patterns directly viewed by an operator through an eye piece; or (iii) G_{M1} patterns discerned by flow cytometry.

For the purposes of this application "insemination" is understood to have a meaning dependent upon the reproductive approach. For example, for "intercourse," insemination is understood to mean introduction of sperm into a female's reproductive tract. For example, for "intracervical insemination (ICI)," insemination is understood to mean introduction of sperm into a female's cervix. For "intrauterine insemination (ILTI)," insemination is understood to mean when sperm is introduced into a female's uterus. For *"in vitro* fertilization (IVF)," insemination is understood to mean when sperm are introduced into a droplet of medium containing egg cells (oocytes) to allow co-incubation of sperm and egg cell(s). For "pre-capacitating sperm prior to *in vitro* fertilization," insemination is understood to mean when sperm are introduced into a droplet of medium containing egg cells (oocytes) to allow co-incubation of sperm and egg cell(s). For "intracytoplasmic sperm injection (ICSI)," insemination is understood to mean injection of sperm or pre-capacitated sperm into an egg cell. For "gamete intra-fallopian transfer (GIFT)," insemination is understood to mean injection of sperm or pre-capacitated sperm and egg cell(s) into the female's Fallopian tubes. For "subzonal insemination (SUZI)," insemination is understood to mean injection of a single sperm cell or a single pre-capacitated sperm cell just beneath the zona pellucida. For purposes of this application, the term "cryopreservation" refers to the entire process of freezing, storing, and thawing the cells for use

As used herein below, the male is a mammal. In an embodiment, the male is a human. In another embodiment, the male is a non-human mammal. In one such embodiment, the male is a companion animal. In another embodiment, the male is an agricultural animal. In one such embodiment, the male is a canine, feline, equine, bovine, sheep, goat, pig, camellid, or buffalo.

The present invention is as set out in the claims.

In an embodiment, the disclosure provides for a method to identify an approach for achieving mammalian fertilization. A sample of t₀*-in vitro* capacitated sperm cells is treated with a fluorescence label and a sample of t₁*-in vitro* capacitated sperm cells is treated with a fluorescence label. One or more t₀-fluorescence images is obtained, the t₀-fluorescence images displaying one or more G_{M1} localization patterns associated with t₀-fluorescence labeled *in vitro* capacitated sperm cells. And one or more t₁-fluorescence images is obtained, the t₁-fluorescence displaying one or more G_{M1} localization patterns associated with t₁-fluorescence labeled *in vitro* capacitated sperm cells. The t₀-fluorescence images are obtained at post *in vitro* capacitation times selected from: 0.5 hours to 3 hours; 3 hours to 12 hours; 6 hours to 12 hours; 3 hours to 24 hours; 12 hours to 24 hours; or 18 hours to 24 hours (t₀); and the t₁-fluorescence images being obtained at post capacitation time t₁ wherein t₁ is greater than t₀. A number of apical acrosome (AA) G_{M1} localization patterns, a number of acrosomal plasma membrane (APM) G_{M1} localization patterns and a total number of G_{M1} localization patterns are measured for the t₀-fluorescence labeled *in vitro* capacitated sperm cells displayed in the t₀-fluorescence images to determine a percentage of t₀-[AA G_{M1} localization patterns plus APM G_{M1} localization patterns]. And a number of apical acrosome (AA) G_{M1} localization patterns, a number of acrosomal plasma membrane (APM) G_{M1} localization patterns and a total number of G_{M1} localization patterns are measured for the t₁-fluorescence labeled *in vitro* capacitated sperm cells displayed in the ti-fluorescence images to determine a percentage of ti-[AA G_{M1} localization patterns plus APM G_{M1} localization patterns]. The percentage of ti-[AA G_{M1} localization patterns plus APM G_{M1} localization patterns] is compared to the percentage of t₀-[AA G_{M1} localization patterns plus APM G_{M1} localization patterns] to determine an *in vivo* capacitation time selected from a late *in vivo* capacitation time greater than 12 hours or a standard *in vivo* capacitation time of 12 hours or less. A difference in percentage of t₀-[AA G_{M1} localization patterns plus APM G_{M1} localization patterns] and the percentage of ti-[AA G_{M1} localization patterns plus APM G_{M1} localization patterns] corresponding to: greater than one standard deviation from a standard of 35 % indicates a late *in vivo* capacitation time greater than 12 hours; or less than one standard deviation from a standard of 35 % indicates a standard *in vivo* capacitation time less than 12 hours. A reference percentage of [AA G_{M1} localization patterns plus APM G_{M1} localization patterns] corresponding to: greater than 35 % indicates a high fertility status; one standard deviation below 35 % indicates a medium fertility status; and two or more standard deviations below 35 % indicates a low fertility status. Further when a difference in percentage of t₀-[AA G_{M1} localization patterns plus APM G_{M1} localization patterns] and the percentage of ti-[AA G_{M1} localization patterns plus APM G_{M1} localization patterns] corresponding to: greater than one standard deviation from a standard of 35 %, then a ti-fertility status is determined based on a comparison of the percentage of measured ti-[AA G_{M1} localization patterns plus APM G_{M1} localization patterns] to the reference percentage of [AA G_{M1} localization patterns plus APM G_{M1} localization patterns]; or less than one standard deviation from a standard of 35 % then a ti-fertility status is determined based on a comparison of the reference percentage of [AA G_{M1} localization patterns plus APM G_{M1} localization patterns] to the percentage of measured ti-[AA G_{M1} localization patterns plus APM G_{M1} localization patterns] or the percentage of measured t₀-[AA G_{M1} localization patterns plus APM G_{M1} localization patterns]. Based on the male's ti-fertility status and *in vivo* capacitation time, a time period for insemination and a reproductive approach are identified to use in order to achieve fertilization.

In an instance of the foregoing embodiment, wherein the male has at least normal sperm concentration and late *in vivo* capacitation time: (i) the reproductive approach for high ti-fertility status is selected from the group consisting of: modifying the timing of intercourse to late *in vivo* capacitation time; modifying the timing of intracervical insemination (ICI) to late *in vivo* capacitation time; modifying the timing of intrauterine insemination (IUI) to late *in vivo* capacitation time; pre-capacitating sperm prior to intracervical insemination, or pre-capacitating sperm prior to intrauterine insemination; (ii) the reproductive approach for medium ti-fertility status is selected from group consisting of: modifying the timing of intracervical insemination (ICI) to late *in vivo* capacitation time; modifying the timing of intrauterine insemination (IUI) to late *in vivo* capacitation time; modifying the timing of *in vitro* fertilization (IVF) to late *in vivo* capacitation time; pre-capacitating sperm prior to intracervical insemination; intrauterine insemination (IUI); pre-capacitating sperm prior to intrauterine insemination; or pre-capacitating sperm prior to *in vitro* fertilization, or (iii) the reproductive approach for low ti-fertility status is selected from group consisting of: modifying the timing of *in vitro* fertilization (IVF) to late *in vivo* capacitation time; modifying the timing of intracytoplasmic sperm injection (ICSI) to late *in vivo* capacitation time; modifying the timing of gamete intra-fallopian transfer (GIFT) to late *in vivo* capacitation time; modifying the timing of subzonal insemination (SUZI) to late *in vivo* capacitation time; pre-capacitating sperm prior to *in vitro* fertilization, pre-capacitating sperm prior to intracytoplasmic sperm injection, pre-capacitating sperm prior to gamete intra-fallopian transfer, or pre-capacitating sperm prior to subzonal insemination.

In another instance of the foregoing embodiment, wherein the male has a less than normal sperm concentration and late *in vivo* capacitation time: (i) the reproductive approach for ti-high fertility status is selected from the group consisting of: modifying the timing of intercourse to late *in vivo* capacitation time; modifying the timing of intracervical insemination (ICI) to late *in vivo* capacitation time; modifying the timing of intrauterine insemination (IUI) to late *in vivo* capacitation time; pre-capacitating sperm prior to intracervical insemination (ICI), or pre-capacitating sperm prior to intrauterine insemination (IUI); (ii) the reproductive approach for ti-medium fertility status is selected from group consisting of: modifying the timing of intracervical insemination (ICI) to late *in vivo* capacitation time; modifying the timing of intrauterine insemination (IUI) to late *in vivo* capacitation time; modifying the timing of *in vitro* fertilization (IVF) to late *in vivo* capacitation time; pre-capacitating sperm prior to intracervical insemination, pre-capacitating sperm prior to intrauterine insemination; or pre-capacitating sperm prior to *in vitro* fertilization; or (iii) the reproductive approach for ti-low fertility status is selected from group consisting of: modifying the timing of *in vitro* fertilization (IVF) to late *in vivo* capacitation time; intracytoplasmic sperm injection (ICSI), modifying the timing of intracytoplasmic sperm injection (ICSI) to late *in vivo* capacitation time; modifying the timing of gamete intra-fallopian transfer (GIFT) to late *in vivo* capacitation time; modifying the timing of subzonal insemination (SUZI) to late *in vivo* capacitation time, pre-capacitating sperm prior to *in vitro* fertilization, pre-capacitating sperm prior to intracytoplasmic sperm injection, pre-capacitating sperm prior to gamete intra-fallopian transfer, or pre-capacitating sperm prior to subzonal insemination.

In another instance of the foregoing embodiment, wherein the male has at least normal sperm concentration and standard *in vivo* capacitation time: (i) the reproductive approach for high ti-fertility status is selected from the group consisting of: intercourse, intracervical insemination (ICI), pre-capacitating sperm prior to intracervical insemination, intrauterine insemination (IUI), or pre-capacitating sperm prior to intrauterine insemination; (ii) the reproductive approach for medium ti-fertility status is selected from the group consisting of: intracervical insemination (ICI), pre-capacitating sperm prior to intracervical insemination; intrauterine insemination (IUI); pre-capacitating sperm prior to intrauterine insemination; or *in vitro* fertilization (IVF) or pre-capacitating sperm prior to *in vitro* fertilization; or (iii) the reproductive approach for low ti-fertility status is selected from group consisting of: *in vitro* fertilization (IVF), pre-capacitating sperm prior to *in vitro* fertilization, intracytoplasmic sperm injection (ICSI), pre-capacitating sperm prior to intracytoplasmic sperm injection, gamete intra-fallopian transfer (GIFT), pre-capacitating sperm prior to gamete intra-fallopian transfer, subzonal insemination (SUZI), or pre-capacitating sperm prior to subzonal insemination.

In another instance of the foregoing embodiment, wherein the male has a less than normal sperm concentration and standard *in vivo* capacitation time: (i) the reproductive approach for high ti-fertility status is selected from group consisting of: intracervical insemination (ICI), pre-capacitating sperm prior to intracervical insemination, intrauterine insemination (IUI) or pre-capacitating sperm prior to intrauterine insemination; (ii) the reproductive approach for medium ti-fertility status is selected from group consisting of: intracervical insemination (ICI), pre-capacitating sperm prior to intracervical insemination, intrauterine insemination (IUI) or pre-capacitating sperm prior to intrauterine insemination; *in vitro* fertilization (IVF), pre-capacitating sperm prior to *in vitro* fertilization, or (iii) the reproductive approach for low ti-fertility status is selected from group consisting of: *in vitro* fertilization (IVF), pre-capacitating sperm prior to *in vitro* fertilization, intracytoplasmic sperm injection (ICSI), pre-capacitating sperm prior to intracytoplasmic sperm injection, gamete intra-fallopian transfer (GIFT), pre-capacitating sperm prior to gamete intra-fallopian transfer, subzonal insemination (SUZI) or pre-capacitating sperm prior to subzonal insemination.

In an instance of each of the foregoing embodiments, the identifying step is also based on one or more of the following: patient demographics, reproductive status of female partner, sperm concentration, total motility, progressive motility, semen volume, semen pH, semen viscosity and/or sperm morphology and combinations thereof.

In an instance of each of the foregoing embodiments, the *in vitro* capacitated sperm cells are treated with a fixative for a fixative time period of: at least 0.5 hour; at least 3 hours; at least 12 hours; at least 18 hours; at least 24 hours; at least 30 hours; at least 36 hours; or at least 48 hours, for a fixation time period ranging between 0.5 hours to 3 hours; 3 hours to 12 hours; 6 hours to 12 hours; 3 hours to 18 hours; 6-18 hours; 6-24 hours; 12 hours to 24 hours; 18 hours to 24 hours; 18-30 hours; 18-36 hours; 24-30 hours; 24-26 hours; 18-48 hours; 24-48 hours; or 36-48 hours.

In an instance of each of the foregoing embodiments, the sperm cells were treated to cryopreservation procedures and stored prior to being treated *in vitro* with capacitation conditions.

In one embodiment, capacitation conditions include *in vitro* exposure to 2-hydroxypropyl-β-cyclodextrin. In one embodiment, the fixative includes paraformaldehyde or glutaraldehyde.

The male's fertility status data may be compared to data of known male fertility status which is associated with a known time period for insemination and associated with a known reproductive approach. In such embodiments, the known fertility status includes: fertile with sperm capacitation within 3 hours; fertile with sperm capacitation within 12 hours, fertile with capacitation between 12 and 24 hours; and non-fertile.

In the foregoing embodiments, the reproductive approach may correspond to natural insemination approaches and artificial insemination approaches as known in the art. In one embodiment, the reproductive approach includes: intercourse; intracervical insemination (ICI), intrauterine insemination (IUI), *in vitro* fertilization (IVF), intracytoplasmic sperm injection (ICSI), pre-capacitating sperm prior to *in vitro* fertilization, gamete intra-fallopian transfer (GIFT), and subzonal insemination (SUZI).

For embodiments where the reproductive approach corresponds to intercourse, the time period for intercourse is determined relative to the female's timing of ovulation, as visualized with ultrasonography, and/or predicted based on timing of the menstrual cycle, use of ovulation timing kits, changes in body temperature, or timing relative to one or more injections with one or more hormones designed to induce follicular growth and ovulation. For example, the insemination time period may correspond to: 96 hours before the time of ovulation; 72 hours before the time of ovulation; 48 hours before the time of ovulation; 24 hours before the time of ovulation; 12 hours before the time of ovulation; 6 hours before the time of ovulation; or at the time of ovulation.

For embodiments where the reproductive approach corresponds to ICI or IUI, the time period for insemination is determined relative to the female's timing of ovulation, as visualized with ultrasonography, and/or predicted based on timing of the menstrual cycle, use of ovulation timing kits, changes in body temperature, or timing relative to one or more injections with one or more hormones designed to induce follicular growth and ovulation. For example, the insemination time period may correspond to: 96 hours before the time of ovulation; 72 hours before the time of ovulation; 48 hours before the time of ovulation; 24 hours before the time of ovulation; 12 hours before the time of ovulation; 6 hours before the time of ovulation; or at the time of ovulation.

For embodiments where the reproductive approach corresponds to IVF, the time period for insemination corresponds to 3 hours before determination of pronuclear formation; 4 hours before determination of pronuclear formation; 6 hours before determination of pronuclear formation; 12 hours before determination of pronuclear formation; 18 hours before determination of pronuclear formation; 24 hours before determination of pronuclear formation; or 30 hours before determination of pronuclear formation.

For embodiments where the reproductive approach corresponds to pre-capacitating sperm prior to *in vitro* fertilization, the time period for pre-capacitation corresponds to incubating sperm in media containing one or more stimuli for capacitation, for periods of 24 hours before insemination; 18 hours before insemination; 12 hours before insemination; 6 hours before insemination; 4 hours before insemination; 3 hours before insemination; or 1 hour before insemination.

For embodiments where the reproductive approach corresponds to intracytoplasmic sperm injection (ICSI), the time period for pre-capacitation prior to insemination corresponds to incubating sperm in media containing one or more stimuli for capacitation, for periods of 24 hours before insemination; 18 hours before insemination; 12 hours before insemination; 6 hours before insemination; 4 hours before insemination; 3 hours before insemination; or 1 hour before insemination.

For embodiments where the reproductive approach corresponds to gamete intra-fallopian transfer (GIFT), the time period for pre-capacitation prior to insemination corresponds to incubating sperm in media containing one or more stimuli for capacitation, for periods of 24 hours before insemination; 18 hours before insemination; 12 hours before insemination; 6 hours before insemination; 4 hours before insemination; 3 hours before insemination; or 1 hour before insemination.

For embodiments where the reproductive approach corresponds to subzonal insemination (SUZI), the time period for pre-capacitation prior to insemination corresponds to incubating sperm in media containing one or more stimuli for capacitation, for periods of 24 hours before insemination; 18 hours before insemination; 12 hours before insemination; 6 hours before insemination; 4 hours before insemination; 3 hours before insemination; or 1 hour before insemination.

In some instances of the foregoing embodiments, other parameters may be used to identify a time period for insemination and a reproductive approach. The other parameters may include one or more of the following: patient demographics, reproductive status of female partner, sperm concentration, total motility, progressive motility, semen volume, semen pH, semen viscosity and/or sperm morphology and combinations thereof.

In an instance of any of the foregoing embodiments, a semen sample is processed using a wide orifice pipette having an orifice of sufficient size in diameter to prevent shearing of a sperm membrane and the semen sample is processed without use of a reagent that can damage sperm membranes. In one such embodiment, the processed semen sample is exposed to capacitating media, fixative, and reagents for determining G_{M1} localization patterns.

In an embodiment, a reagent that can damage sperm membranes is selected from the group consisting of: (i) a protease; (ii) a nuclease (iii) a mucolytic agent; (iv) a lipase; (v) an esterase and (vi) Glycoside hydrolases. Examples of compounds which may similarly interfere with the ability of sperm to respond to capacitation stimuli include: (i) a protease, including but not limited to, chymotrypsin, trypsin, collagenase, bromelain; (ii) a nucleases, including but not limited to, Dornase, HindIII, EcoRI; (iii) a mucolytic agent, including but not limited to, Erdostein, Acetylcysteine, Guiafenesin; (iv) a lipase, including but not limited to, Phospholipase A1, Phospholipase C, Lipoprotein lipase; (v) an esterase, including but not limited to, Cholinesterase, Thioesterase, Alkaline phosphatase; and (vi) Glycoside hydrolases, including but not limited to, Alpha-amylase, beta-galactosidase, hyaluronidase, neuorminodases, and lysozyme.

The population size for one or more G_{M1} labeled localization patterns may be determined, such that the percent change about Cap-Score is minimized within an individual. The one or more G_{M1} labeled localization patterns may comprise AA G_{M1} localization pattern, APM G_{M1} localization pattern, Lined-Cell G_{M1} localization pattern, intermediate (INTER) G_{M1} localization pattern, post acrosomal plasma membrane (PAPM) G_{M1} localization pattern, apical acrosome/post acrosome (AA/PA) G_{M1} localization pattern, equatorial segment (ES) G_{M1} localization pattern, and diffuse (DIFF) G_{M1} localization pattern.

### EXAMPLES

### Example 1

### Sperm Handling Methods

Three common methods to reduce viscosity were evaluated. Ejaculates were: 1) Incubated for 0.25, 1.25 or 2 hours, 2) diluted 1:1 with Modified Human Tubal Fluid (Irvine Scientific; Santa Anna, CA) and then passed through a wide orifice transfer pipette ("WOTP") or a Pasteur pipette ("PP"), 3) Enzymatically digested with chymotrypsin ("chymo"). Pilot studies revealed that passage through a hypodermic needle negatively affected motility and membrane integrity and was not studied further. After liquefaction, samples were washed and incubated under capacitating (CAP) and non-capacitating (NC) conditions. Cap-Score values were obtained via fluorescence microscopy according to the calculation described above.

### Reliability and Reproducibility of Cap-Score^{™}

Following liquefaction of semen samples from consenting men, sperm were washed, incubated, fixed and then evaluated via fluorescence microscopy for G_{M1} localization patterns. Precision of scoring within one sample, and variation between readers scoring the same samples were both assessed. Student's t-Test employing unequal variance was done using Microsoft Excel (2013).

### Cap-Score Correlation with Traditional Semen Analysis Parameters

Semen samples from consenting patients were liquefied, washed and incubated under both non-capacitating and capacitating conditions. Semen analysis was performed according to WHO guidelines. Cap-Score values were obtained via fluorescence microscopy. Statistical analyses were done using Microsoft Excel (2013) and XLSTAT (2015).

### Assessment of Capacitation using Cap-Score^{™}For Males of Demonstrated Fertility Compared to Males Being Assessed for Fertility

The Cap-Score values were determined on consenting men from two cohorts: 1) known fertility (pregnant partner or fathering a child less than 3 years old), and 2) patients seeking their first semen analysis. Following liquefaction, sperm were washed and 3 million incubated for 3 hour under non-capacitating (NC) and capacitating (CAP) conditions. Sperm were fixed overnight and G_{M1} localization patterns assessed via fluorescence microscopy.

### RESULTS

Liquefaction time, dilution and pipetting did not alter Cap-Score values. Control (incubation only), WOTP and PP treated samples had Cap-Score values of 41±4, 40±5, and 41±6 (n=5; CAP). A decrease in response to capacitating stimuli was observed when samples were liquefied using chymo (P=0.03). Control samples had Cap-Score values of 40±6 (n=5; CAP) whereas samples enzymatically liquefied had Cap-Score values of 31±4 (n=5; CAP). Because chymo is a protease that can cut membrane proteins, it was examined to determine if the reduced Cap-Score value resulted from an alteration in labeling. Samples not exposed to capacitation stimuli were compared and no difference was observed. Control and enzymatically liquefied samples had Cap-Score values of 22±4 and 21±5 (n=5; NC). These data support the view that treating semen with chymo, although widely used in clinical practice, can inhibit the ability of sperm to respond to capacitation stimuli.

Classes of compounds which may similarly interfere with the ability of sperm to respond to capacitation stimuli include: (i) a protease, including but not limited to, chymotrypsin, trypsin, collagenase, bromelain; (ii) a nucleases, including but not limited to, Dornase, HindIII, EcoRI; (iii) a mucolytic agent, including but not limited to, Erdostein, Acetylcysteine, Guiafenesin; (iv) a lipase, including but not limited to, Phospholipase A1, Phospholipase C, Lipoprotein lipase; (v) an esterase, including but not limited to, Cholinesterase, Thioesterase, Alkaline phosphatase; and (vi) Glycoside hydrolases, including but not limited to, Alpha-amylase, beta-galactosidase, hyaluronidase, neuorminodases, and lysozyme.

Liquefaction times of up to 2 hours and mechanical liquefaction using WOTP and/or PP did not influence capacitation. In contrast, the use of enzymes such as chymo reduced the ability of sperm to capacitate, as measured by Cap-Score^{™} Test. These results demonstrate the importance of knowing how semen processing methods impact sperm function.

Precision was evaluated by comparing the percent change about Cap-Score values (%Δ=(y₂-y₁)/y₂) when 50, 100, 150 and 200 sperm were evaluated. Changes in values of 11, 6 and 5% were observed for each addition of 50 sperm (n≥23). To be conservative, Cap-Score value was determined by counting the G_{M1} localization patterns of at least 150 cells. To assess variation within and between readers, 8 large image files containing up to 5,000 sperm were generated by combining images taken from multiple visual fields. Two different readers were trained and they determined Cap-Score values by randomly resampling each image 20 times, counting 150 cells each time. When scoring the same sample, individual readers reported an average SD of three (3) Cap-Score units. The difference between readers when scoring the same sample ranged from 0.00 to 1.52, with an average difference of one (1) between the readers for any given sample. Applying the Bonferroni correction for multiple comparisons, no difference between readers was observed for any image file (p-values ranged from 0.02 to 0.99).

Cap-Score was not affected by liquefaction time or mechanical liquefaction with WOTP or PP (n=5), though after washing and incubation, samples that had undergone two (2) hours liquefaction or passage through a PP showed a greater decline in motility (p=0.02, 3E-3). Use of a needle damaged sperm and was not investigated further. Liquefaction with chymo reduced Cap-Scores in CAP (p=0.03), but not NC samples (p=0.74). Samples incubated with chymo (n=5; 3mg/ml) could not be scored due to membrane damage, yet showed an increase in motility under NC conditions (p=9E-4).

### Example 2

This example was conducted using a cohort comparison between fertile (cohort 1, pregnant or recent father) and potential subfertile/infertile men (cohort 2, men questioning fertility). Relationships between Cap-Score and traditional semen measures were also explored.

All studies approved by WIRB (20152233). Semen samples were liquefied, washed, and incubated under non-capacitating and capacitating conditions. Sperm were fixed overnight and Cap-Score determined via fluorescence microscopy. Semen quality measures were evaluated according to WHO. T-Test, ANOVA and correlation analyses were done using Microsoft Excel (2013) and XLSTAT.

The mean Cap-Score for cohort 1 was 35.3 (SD= 7.7%; n=76 donors; 187 collections). Cap-Scores were lower for cohort 2 (p=1.0E-03), with 33.6% (41/122) having Cap-Scores below one (1) SD below the mean for cohort 1, versus an expected 16%. For cohort 2, no relationship was observed between Cap-Score and morphology (p=0.28), motility (p=0.14) or concentration (p=0.67). 93.4% (114/122) of men in cohort 2 exhibited normal motility, yet 30.7% (35/114) of them had Cap-Scores below one (1) SD below the mean. Similarly, 101 of 122 men (82.7%) exhibited normal concentration with 32.6% (33/101) having Cap-Scores below one (1) SD below the mean. These results show that capacitation defects are common in men having difficulty conceiving and the Cap-Score provides functional data that complement semen analysis.

The ability of sperm to capacitate differs between fertile men and those having trouble conceiving. Because capacitation is required for fertilization, the Cap-Score can provide an important functional complement to standard semen analysis and may help in choosing the most appropriate fertility treatment.

Common measures of semen quality are subjective and can vary within and among readers, making the assessment of male fertility challenging. The Cap-Score^{™} Test evaluates the ability of sperm to capacitate, a necessity for male fertility. The data presented here show that the Cap-Score^{™} Test is highly reproducible and reliable within and between readers, which are key considerations when attempting to diagnose male infertility.

The Cap-Score mean (µ=39) and SD (σ=7) from 41 fertile men were used to estimate the number of known fertile men needed for establishing a robust fertile capacitation profile. For a power analysis, an acceptable range about the mean was set at 3% and a two-tailed t-test at the p<0.01 level, with a probability of detecting a difference this large of 90% were applied. Results suggested that a valid standard can be established with ≥85 individuals. A preliminary normal fertile standard was created using 125 observations from 41 unique donors. The Cap-Score values were averaged by donor and then converted to z-scores ((X-µ)/σ; X=observation, µ=39; σ=7). This transformed the µ to 0 and the σ to 1, with converted values representing the distance from the µ (mean) in units of σ (S.D.). The normal fertile standard was tested against Cap-Score values from 93 men seeking fertility exams. This cohort scored significantly below the fertile population (p=1.6E-5), with 27 and 38% having z-scores ≤-2 and between -1 and -2. Only 35% scored near or above the mean. These data suggest that, in comparison to fertile men, many men seeking fertility exams have defects in capacitation.

### Example 3

The procedures used in Examples 1 and 2 were used in Example 3.

Classic semen analyses provide little information on the ability of samples to fertilize and egg. Capacitation is required for fertilization and can be assessed using G_{M1} localization. A comparison of the Cap-Score values from two cohorts of men revealed significant differences in their ability to capacitate. A robust capacitation profile can be defined and employed for identifying abnormalities. Remarkably, 33% of men questioning their fertility had z-scores ≤-1, versus an expected result of 16%. Combining the Cap-Score^{™} Test with traditional analyses should prove valuable in diagnosing male infertility.

Samples from 122 men referred to an infertility specialist were analyzed and had Cap-Scores ranging from 13 to 52%. An analysis of variance was done to compare Cap-Score values and sperm morphology. Samples were classified as having 0, 1, 2, 3, or ≥ 4% normal forms (scores ≥ 4% are considered normal, WHO) and mean Cap-Scores were compared among the groups. No relationship between Cap-Score value and morphology was observed (P=0.67). Next, sperm concentration (range 3×10⁶ to 210×10⁶/mL) was compared to Cap-Score value using the Pearson product-moment correlation coefficient and no connection was found (r=0.01, P=0.90). Lastly, Cap-Score value was compared to total % motility (range 15 to 80%) and the two measures were determined to be independent (r=0.14 P=0.21). Multiple donors who were classified as normal by WHO criteria had Cap-Score values more than two (2) SD below the normal mean, supporting the view that even normal appearing sperm can have functional abnormalities.

Traditional semen analysis identifies only 50% of male infertility cases. This study shows that there is little relationship between Cap-Score value and standard semen analysis parameters. Since capacitation is necessary for fertilization, the addition of the Cap-Score test to traditional semen evaluations could both identify cases of idiopathic infertility and help clinicians counsel couples towards the most appropriate treatment

The data presented herein demonstrate that a male's Cap-Score value may provide guidance on an appropriate mechanism for achieving successful mammalian pregnancies, including recommended assisted reproductive technology such as *in vitro* fertilization (IVF), or intracytoplasmic sperm injection (ICSI). The male may be a human or a non-human mammal. The Cap-Score value in combination with other components of a semen analysis, including concentration, total motility, progressive motility, volume, pH, viscosity and/or morphology may be considered. For example, the recommended assisted reproductive technology for two males with the same Cap-Score may differ if their sperm counts or sperm motility differ. In addition, the fertility status or reproductive health of the female partner would also be considered by the clinician.

### Example 4

Semen samples from consenting men were liquefied, washed and aliquots incubated under non-capacitating (NC) or capacitating (CAP) conditions. The consenting men included men who were classified as fertile based on a pregnant partner or the male being a recent biological father. The consenting men also included men seeking fertility exams. Capacitation conditions include *in vitro* exposure to 2-hydroxypropyl-β-cyclodextrin. Non-capacitation conditions include lack of *in vitro* exposure to any of bicarbonate ions, calcium ions and a mediator of sterol efflux such as 2-hydroxypropyl-β-cyclodextrin for varying periods of time. The *in vitro* capacitated sperm and the *in vitro* non-capacitated sperm were then fixed in a fixative such as paraformaldehyde or glutaraldehyde. The fixed *in vitro* capacitated sperm and the *in vitro* non-capacitated sperm were then labeled with a fluorescent labeled cholera toxin b subunit.

For one dataset, the sperm samples were incubated in capacitation or non-capacitation conditions for three (3) hours, fixed, labeled and then analyzed ("day0"). For a second dataset, the sperm samples were incubated in capacitation or non-capacitation conditions for three (3) hours, fixed overnight, labeled and then analyzed ("day1"). For a third dataset, the sperm samples were incubated in capacitation or non-capacitation conditions for three (3) hours, fixed, labeled and then analyzed. For a third dataset, the sperm samples were incubated in capacitation or non-capacitation conditions for 24 hours, fixed, labeled and then analyzed ("24hrCap"). Sperm capacitation was assessed using localization of G_{M1} (Cap-Score^{™}).

102 sperm samples from 36 fertile men were evaluated at day0 and day1. Between day0 and day1, an increase in Cap-Score was observed in 81% (83/102) of sperm samples, with 44% of the sperm samples (45/102) having an increase in in Cap-Score of more than one (1) standard deviation (7%).

Sperm samples from 17 men seeking fertility treatment were evaluated at day0 and day1. Between day0 and day1, an increase in Cap-Score was observed in 29% (5/17) where the Cap-Scores increased more than one (1) standard deviation (7%).

To determine whether this change in Cap-Score was physiological or an artifact of being in fixative overnight, semen samples from nine (9) fertile men were analyzed at day0, day1 and after 24 hours of *in vitro* incubation in capacitation or non-capacitation medium and then fixed. All *in vitro* non-capacitated samples were equivalent (Cap-Scores of 19±2, 23±2 and 20±1%) and were different from the *in vitro* capacitated samples (Cap-Scores of 28±1, 34±2 and 31±2%, respectively). The Cap-Scores on day1 were significantly greater than the Cap-Scores for day0 (p=0.03). However, the Cap-Scores for *in vitro* capacitated samples incubated overnight in the fixative (day1) or in capacitating medium (24hrCap) were the same (p=0.33).

Consistent with prior literature, these data show that sperm membrane changes involved in capacitation still occur over time in certain fixatives. These data suggest that sperm achieve capacitation at different times in different ejaculates. To see if this was reproducible for an individual, 91 samples from 25 fertile men were classified as either early or late capacitators (day1-day0>7). The average concordance of change within donors was 76%, showing that capacitation timing was highly consistent within men.

### Example 5

Semen samples from 8 fertile men (pregnant partner or recent father) were used to examine the effect of cryopreservation on capacitation timing. Liquefied ejaculates were split; half processed immediately (fresh) and the other cryopreserved in test yolk buffer with glycerol (Irvine Scientific). Cryopreserved samples were subsequently thawed and processed (CryoT). Fresh and CryoT aliquots were washed and then incubated under non-capacitating (NC) and capacitating (CAP) conditions for 3 hours. Capacitation timing differs among men and can be evaluated by comparing Cap-Score differences from day 1 (after overnight incubation under conditions that promote/allow capacitation) to day 0 (analyzed after 3 hours incubation).

Cap-Score increased in NC CryoT treatments for both day 0 and day 1 when compared to fresh. For day 0 there was a 155% increase ((fresh - CryoT)/fresh; 11±1.6% vs 28±2.4%; n=7; p=0.00) and a 79% increase for day 1 (19±2.6% vs 34±2.7%; n=8; p=0.00). Conversely, Cap-Score for CAP treatments remained the same for both day 0 (26±3.1% vs 30±2.5%; n=7; p=0.31,) and day 1 (34±2.9% vs 34±1.7%; n=8; p=0.86,). Average post-thaw and post wash motilities of 27±3.5% and 31±8.6% for CryoT samples suggest reasonable post-thaw viability. When samples for day 0 and day 1 were compared, no difference in capacitation timing (day 1 - day 0; n=7) was observed between fresh and CryoT samples for NC (10±2.2% vs 8±2.7%) or CAP treatments (8±3.8% vs 5±2.3%).

### Example 6

All procedures, for specimen collection, were approved by WIRB (Protocol #20152233). Semen samples were collected from consenting men by manual masturbation after a minimum of 2 and a maximum of 5 days of sexual abstinence. Those samples having fewer than 10×10⁶ motile sperm cells were discarded from this study.

Ejaculates were liquefied at 37°C for at least 15 minutes and for no more than two (2) hours. Subsequent to liquefaction, the sperm were removed from the seminal plasma by centrifugation through Enhance S-Plus Cell Isolation Media (Vitrolife, reference: 15232 ESP-100-90%) at 300xg for 10 minutes. The cells were collected, resuspended in ~4ml of Modified Human Tubal Fluid medium (mHTF; Irvine Scientific; reference 90126) and pelleted at 600xg for 10 minutes. The sperm were resuspended in mHTF with and without capacitation stimuli and incubated for three (3) hours. Following incubation, the samples were fixed as described (Selvaraj V, et al., "Segregation of micron-scale membrane sub-domains in live murine sperm," J Cell Physiol. 206: 636-46 (2006)) with paraformaldehyde (Electron Microscopy Sciences; Hatfield, PA) for at least 30 minutes prior to labeling.

Samples were labeled with 2 µg/mL of Cholera Toxin B, conjugated with Alexa Fluor 488 (CTB; Thermo Fisher: C34775). After ten minutes, 5 µl of the labeled sperm were placed on a microscope slide, overlaid with a cover slip and moved to an imaging station.

Imaging stations consisted of Nikon Eclipse NI-E microscopes equipped with: CFI60 Plan Apochromat Lambda 40x Objectives, C-FL AT GFP/FITC Long Pass Filter Sets, Hamamatsu ORCA-Flash 4.0 cameras, H101F - ProScan III Open Frame Upright Motorized H101F Flat Top Microscope Stages, and 64bit imaging workstations running NIS Elements software (Nikon; Melville, NY). These systems were programmed to automatically capture sets of 15x15 stitched images containing up to 5,000 sperm.

The Cap-Score SFT detects and analyzes localization patterns of the ganglioside G_{M1}. Two independent readers were trained to identify G_{M1} localization patterns that have been associated with capacitation of human sperm. The proportion of sperm within a sample having undergone capacitation was determined and reported as the Cap-Score.

Power Analysis was done using G*power (Faul et al., "G* Power 3: A flexible statistical power analysis program for the social, behavioral, and biomedical sciences," Behav Res Methods. 39: 175-91 (2007)). Student's t-Test was done using Microsoft Excel (2013). Linear Regression and Bartlett's test of homoscedasticity were carried out in XLSTAT Version 2015.5.01.22912.

### RESULTS

The first step in determining Cap-Score precision was to define the number of cells to count per sample. In general, as the number of cells counted increases, there is an increase in precision. However, at some point counting additional cells becomes redundant, as the Cap-Score will not change with additional observations. To identify the point when counting additional cells is unnecessary, the percent change about Cap-Score when 50, 100, 150 and 200 sperm was evaluated (Table 1).

**Table 1: Percent change in Cap-Score with increasing number of counted sperm.**

| **No. of counted sperm** | **Mean % Δ** | **STDEV** | **No. of Obs.** | **SEM** | **95% CI** | |
|---|---|---|---|---|---|---|
| | | | | | **LL** | **UL** |
| from 50 to 100 | 11% | 9% | 23 | 2% | 7% | 14% |
| from 100 to 150 | 6% | 5% | 26 | 1% | 4% | 8% |
| from 150 to 200 | 5% | 3% | 26 | 1% | 4% | 6% |

The percent change in Cap-Score when counting 50 or 100 sperm was large when compared to the percent change when counting 100 or 150 and 150 or 200 sperm. These observations supported the view that Cap-Score precision was only modestly improved by counting more than 100 sperm. However, the 95% confidence intervals for the percent change when counting 50 or 100 and 150 or 200 did not overlap, suggesting a significant reduction in percent change when at least 150 cells were counted. To be conservative, Cap-Score was determined by counting the G_{M1} localization patterns of at least 150 cells.

To further explore Cap-Score reliability and assess its potential as an objective measure of semen quality, its measurement was investigated to determine its accuracy within individual readers. Accuracy is defined as the proximity of measurements to the true value. The true value of an unknown population can be estimated by its central tendency, or the mean. One can judge whether a data set has a strong or a weak central tendency based on its dispersion, or the inverse of precision (JCGM/WG2 2008). That is to say that as dispersion increases, there is a decrease in precision. The standard deviation (SD) and Coefficient of Variation (CoV) measure the amount of dispersion within a sample. A standard deviation close to zero (0) indicates that the data points tend to be clustered tightly about the mean, while a high standard deviation indicates that the data points are spread out. Similarly, the CoV represents the amount of dispersion relative to the mean (CoV=SD/mean) and is useful for comparing the degree of variation from one data series to another, even if the means are drastically different from each other.

Prior to evaluating Cap-Score accuracy within readers, the number of images for each reader to sample was estimated. To this end, two semen donor groups were defined based on a cut-off of one (1) SD below the mean Cap-Score for a population of men with presumed fertility (pregnant wife or child less than 3 years old). The mean Cap-Score for the group with "lower Cap-Scores" was 27 and the "presumed fertile" group was 40. The standard deviations for each group were 5.2 and 4.9 respectively. A power analysis using a two-tailed test was done at the p<0.05 and p<0.01 level, with a probability of detecting a difference this large, if it exists, of 90% (1-beta=0.90). The results of these analyses indicated that sample 10 and 14 images respectively (5 and 7 per group) should be sampled. To be conservative, 10 images, each in the "lower Cap-Score" and "presumed fertile" groups, were generated. Sampling this number of images per group ensured that each was sufficiently interrogated to identify any differences in reproducibility that might occur because of either low- or high-value Cap-Scores.

To evaluate the accuracy of the Cap-Score SFT, two different readers determined Cap-Score by randomly resampling 10 images, that contained up to 5,000 sperm each, from the "lower Cap-Score" and "presumed fertile" groups and resampled 20 times by each reader. The SD and CoV were calculated on a per image basis for each reader. The average SD across images and readers was 3 (Fig 1A) and the average CoV was 13% (Fig 1B). Both the SD and CoV showed a linear relationship to Cap-Score. Thus, while there was greater dispersion associated with reading higher Cap-Scores, this appeared to result from a greater Cap-Score magnitude. These data were consistent with a high degree of accuracy, because when the same population of sperm was randomly resampled by the same or different reader, Cap-Score values were clustered tightly about the true value. Since these measure of variance and (or) dispersion are small and stable, they reveal a high degree of Cap-Score reproducibility within readers.

In general, a distribution can be described using its mean and variance. The mean indicates the location of the distribution, while the variance describes how dispersed the data are. One can envisage two distributions where the means are the same, yet the variances are different. For example, one distribution might resemble a normal bell shape, while the other is flatter having more extreme values. To demonstrate similar Cap-Score distributions between readers, 10 stitched images were obtained each for the "lower Cap-Score" and "presumed fertile" groups. Two different readers determined Cap-Scores by randomly resampling each image 20 times. Since each image file contained several magnitudes more sperm than were being sampled, each random resampling represented a distinct subsample of cells from within an individual ejaculate.

An average difference of one (1) in mean Cap-Score was observed between the readers for the 20 different images (Fig. 3). When the Bonferroni correction was applied, no discernable differences were observed. Similarly, Cap-Score variances were not different between readers (Fig. 3). These data support the view that Cap-Score was reproducible between readers, as independent readers obtained similar Cap-Score distributions when resampling the same population of sperm. Collectively, these data provide strong evidence that the Cap-Score SFT is highly reproducible and reliable within and between readers, which are key considerations when attempting to evaluate male reproductive fitness.

The data presented in the current study demonstrate that the Cap-Score SFT is highly reproducible and reliable within and between readers. The data and image files acquired should serve as a foundation for the continued quality control (QC) and quality assurance (QA) within and among laboratories in the evaluation of Cap-Score. For example, two of the 20 image files, one each from the "lower Cap-Scores" and "presumed fertile" groups could be selected at random and scored each day to demonstrate a reader's daily ability to read Cap-Scores. If values are obtained that are outside of acceptable ranges from the established mean (Westgard et al., "A multi-rule Shewhart chart for quality control in clinical chemistry," Clin Chem. 27: 493-50 (1981), the laboratory director can be consulted for remediation. These data can also be used to track individual readers over time and to identify potential changes in Cap-Score determination. Similarly, as new personal and (or) laboratories are trained and incorporated into the reading rotation, their reading ability can be evaluated by scoring multiple image files and comparing their Cap-Scores to established values. Such an approach would ensure comparable data both within and among laboratories. Only through continued internal and external QA and QC can high standards of sperm function evaluations be maintained.

Male fertility diagnosis has historically been plagued by the inability to assess sperm function; namely, the ability to fertilize (Oehninger et al., "Sperm functional tests," Fertil Steril. 102: 1528-33 (2014); Wang et al., "Limitations of semen analysis as a test of male fertility and anticipated needs from newer tests," Fertil Steril. 102: 1502-07 (2014)). Such a diagnostic capability would provide a functional complement to the descriptive assessments of traditional semen evaluations. Identifying sperm with deficiencies in fertilizing ability may allow for a more specific diagnosis of what is now categorized as idiopathic infertility. Of much greater practical importance, this information could enable a clinician to help counsel a couple toward the most appropriate form of ART to achieve pregnancy. To achieve the previously specified goal, many assays of sperm function have been suggested (e.g., hamster zona penetration assays (Barros et al., "Human Sperm Penetration into Zona-Free Hamster Oocytes as a Test to Evaluate the Sperm Fertilizing Ability," Andrologia. 11: 197-210 (1979); Rogers et al., "Analysis of human spermatozoal fertilizing ability using zona-free ova," Fertil Steril. 32: 664-70 )1979), sperm-ZP binding tests (Liu et al., "Clinical application of sperm-oocyte interaction tests in in vitro fertilization-embryo transfer and intracytoplasmic sperm injection programs," Fertil Steril. 82: 1251-63 (2004), and cervical mucus penetration assays (Alexander, "Evaluation of male infertility with an in vitro cervical mucus penetration test," Fertil Steril. 36: 201-8 (1981); Menge et al., "Interrelationships among semen characteristics, antisperm antibodies, and cervical mucus penetration assays in infertile human couples." Fertil Steril. 51: 486-92 (1989); Eggert-Kruse et al., "Prognostic value of in vitro sperm penetration into hormonally standardized human cervical mucus," Fertil Steril. 51: 317-23 (1989). However, their use has been limited by the great difficulty in obtaining needed materials in a logistically practical fashion. To fill the current void, a diagnostic tool has been developed to evaluate the ability of sperm to undergo the physiological changes required to fertilize an oocyte.

## Claims

1. A method of using a difference in percentage of t₀-[AA G_{M1} localization patterns plus APM G_{M1} localization patterns] and percentage of ti-[AA G_{M1} localization patterns plus APM G_{M1} localization patterns] as an indicator of a time period for insemination and a reproductive approach for achieving mammalian fertilization comprising the steps of:
a. treating a first sample of t₀*-in vitro* capacitated sperm cells with a fixative and a fluorescence label;
b. treating a second sample of t₁*-in vitro* capacitated sperm cells with a fixative and a fluorescence label, wherein the capacitated sperm cells of the first sample and the second sample are treated *in vitro* with capacitation conditions for a capacitation time period ranging between 0.5 hours to 3 hours; 3 hours to 12 hours; 6 hours to 12 hours; 3 hours to 24 hours; 12 hours to 24 hours; or 18 hours to 24 hours;
c. obtaining one or more t₀-fluorescence images displaying one or more G_{M1} localization patterns associated with t₀-fluorescence labeled *in vitro* capacitated sperm cells,
d. obtaining one or more t₁-fluorescence images displaying one or more G_{M1} localization patterns associated with t₁-fluorescence labeled *in vitro* capacitated sperm cells, said t₀-fluorescenceimages and t₁-fluorescence images being obtained at post *in vitro* capacitation times, wherein t₁ is greater than t₀;
e. measuring a number of apical acrosome (AA) G_{M1} localization patterns, a number of acrosomal plasma membrane (APM) G_{M1} localization patterns and a total number of G_{M1} localization patterns for the t₀-fluorescence labeled *in vitro* capacitated sperm cells displayed in the t₀-fluorescence images to determine the percentage of t₀-[AA G_{M1} localization patterns plus APM G_{M1} localization patterns];
f. measuring a number of apical acrosome (AA) G_{M1} localization patterns, a number of acrosomal plasma membrane (APM) G_{M1} localization patterns and a total number of G_{M1} localization patterns for the t₁-fluorescence labeled *in vitro* capacitated sperm cells displayed in the t₁-fluorescence images to determine the percentage of t₁-[AA G_{M1} localization patterns plus APM G_{M1} localization patterns];
g. comparing the percentage of ti-[AA G_{M1} localization patterns plus APM G_{M1} localization patterns] to the percentage of t₀-[AA G_{M1} localization patterns plus APM G_{M1} localization patterns], wherein the comparison indicates an *in vivo* capacitation time selected from a late *in vivo* capacitation time greater than 12 hours or a standard *in vivo* capacitation time of 12 hours or less,
i. wherein a difference in percentage of t₀-[AA G_{M1} localization patterns plus APM G_{M1} localization patterns] and the percentage of ti-[AA G_{M1} localization patterns plus APM G_{M1} localization patterns] corresponding to: greater than one standard deviation from a standard of 35 % indicates a late *in vivo* capacitation time greater than 12 hours; or less than one standard deviation from a standard of 35 % indicates a standard *in vivo* capacitation time less than 12 hours;
ii. wherein a reference percentage of [AA G_{M1} localization patterns plus APM G_{M1} localization patterns] corresponding to: greater than 35 % indicates a high fertility status; one standard deviation below 35 % indicates a medium fertility status; and two or more standard deviations below 35 % indicates a low fertility status;
iii. further wherein a difference in percentage of t₀-[AA G_{M1} localization patterns plus APM G_{M1} localization patterns] and the percentage of ti-[AA G_{M1} localization patterns plus APM G_{M1} localization patterns] corresponding to: greater than one standard deviation from a standard of 35 %, then a ti-fertility status is indicated based on a comparison of the percentage of measured ti-[AA G_{M1} localization patterns plus APM G_{M1} localization patterns] to the reference percentage of [AA G_{M1} localization patterns plus APM G_{M1} localization patterns]; or less than one standard deviation from a standard of 35 % then a ti-fertility status is indicated based on a comparison of the reference percentage of [AA G_{M1} localization patterns plus APM G_{M1} localization patterns] to the percentage of measured ti-[AA G_{M1} localization patterns plus APM G_{M1} localization patterns] or the percentage of measured t₀-[AA G_{M1} localization patterns plus APM G_{M1} localization patterns];
thereby indicating the time period for insemination and the reproductive approach to use in order to achieve fertilization based on the male's ti-fertility status and the *in vivo* capacitation time.

2. The method of claim 1, wherein if the male has at least normal sperm concentration and late *in vivo* capacitation time then
a. the reproductive approach for high ti-fertility status is selected from the group consisting of: modifying the timing of intercourse to late *in vivo* capacitation time; modifying the timing of intracervical insemination (ICI) to late *in vivo* capacitation time; modifying the timing of intrauterine insemination (IUI) to late *in vivo* capacitation time; pre-capacitating sperm prior to intracervical insemination, or pre-capacitating sperm prior to intrauterine insemination;
b. the reproductive approach for medium ti-fertility status is selected from group consisting of: modifying the timing of intracervical insemination (ICI) to late *in vivo* capacitation time; modifying the timing of intrauterine insemination (IUI) to late *in vivo* capacitation time; modifying the timing of *in vitro* fertilization (IVF) to late *in vivo* capacitation time; pre-capacitating sperm prior to intracervical insemination; intrauterine insemination (IUI); pre-capacitating sperm prior to intrauterine insemination; or pre-capacitating sperm prior to *in vitro* fertilization; and,
c. the reproductive approach for low ti-fertility status is selected from group consisting of: modifying the timing of *in vitro* fertilization (IVF) to late *in vivo* capacitation time; modifying the timing of intracytoplasmic sperm injection (ICSI) to late *in vivo* capacitation time; modifying the timing of gamete intra-fallopian transfer (GIFT) to late *in vivo* capacitation time; modifying the timing of subzonal insemination (SUZI) to late *in vivo* capacitation time; pre-capacitating sperm prior to *in vitro* fertilization, pre-capacitating sperm prior to intracytoplasmic sperm injection, pre-capacitating sperm prior to gamete intra-fallopian transfer, or pre-capacitating sperm prior to subzonal insemination.

3. The method of claim 1, wherein if the male has a less than normal sperm concentration and late *in vivo* capacitation time then,
a. the reproductive approach for ti-high fertility status is selected from the group consisting of: modifying the timing of intercourse to late *in vivo* capacitation time; modifying the timing of intracervical insemination (ICI) to late *in vivo* capacitation time; modifying the timing of intrauterine insemination (IUI) to late *in vivo* capacitation time; pre-capacitating sperm prior to intracervical insemination (ICI), or pre-capacitating sperm prior to intrauterine insemination (IUI);
b. the reproductive approach for ti-medium fertility status is selected from group consisting of: modifying the timing of intracervical insemination (ICI) to late *in vivo* capacitation time; modifying the timing of intrauterine insemination (IUI) to late *in vivo* capacitation time; modifying the timing of *in vitro* fertilization (IVF) to late *in vivo* capacitation time; pre-capacitating sperm prior to intracervical insemination, pre-capacitating sperm prior to intrauterine insemination; or pre-capacitating sperm prior to *in vitro* fertilization; and
c. the reproductive approach for ti-low fertility status is selected from group consisting of: modifying the timing of *in vitro* fertilization (IVF) to late *in vivo* capacitation time; intracytoplasmic sperm injection (ICSI), modifying the timing of intracytoplasmic sperm injection (ICSI) to late *in vivo* capacitation time; modifying the timing of gamete intra-fallopian transfer (GIFT) to late *in vivo* capacitation time; modifying the timing of subzonal insemination (SUZI) to late *in vivo* capacitation time, pre-capacitating sperm prior to *in vitro* fertilization, pre-capacitating sperm prior to intracytoplasmic sperm injection, pre-capacitating sperm prior to gamete intra-fallopian transfer, or pre-capacitating sperm prior to subzonal insemination.

4. The method of claim 1, wherein if the male has at least normal sperm concentration and standard *in vivo* capacitation time, then
a. the reproductive approach for high ti-fertility status is selected from the group consisting of: intercourse, intracervical insemination (ICI), pre-capacitating sperm prior to intracervical insemination, intrauterine insemination (IUI), or pre-capacitating sperm prior to intrauterine insemination;
b. the reproductive approach for medium ti-fertility status is selected from the group consisting of: intracervical insemination (ICI), pre-capacitating sperm prior to intracervical insemination; intrauterine insemination (IUI); pre-capacitating sperm prior to intrauterine insemination; or *in vitro* fertilization (IVF) or pre-capacitating sperm prior to *in vitro* fertilization; and
c. the reproductive approach for low ti-fertility status is selected from group consisting of: *in vitro* fertilization (IVF), pre-capacitating sperm prior to *in vitro* fertilization, intracytoplasmic sperm injection (ICSI), pre-capacitating sperm prior to intracytoplasmic sperm injection, gamete intra-fallopian transfer (GIFT), pre-capacitating sperm prior to gamete intra-fallopian transfer, subzonal insemination (SUZI), or pre-capacitating sperm prior to subzonal insemination.

5. The method of claim 1, wherein if the male has a less than normal sperm concentration and standard *in vivo* capacitation time, then
a. the reproductive approach for high ti-fertility status is selected from group consisting of: intracervical insemination (ICI), pre-capacitating sperm prior to intracervical insemination, intrauterine insemination (IUI) or pre-capacitating sperm prior to intrauterine insemination;
b. the reproductive approach for medium ti-fertility status is selected from group consisting of: intracervical insemination (ICI), pre-capacitating sperm prior to intracervical insemination, intrauterine insemination (IUI) or pre-capacitating sperm prior to intrauterine insemination; *in vitro* fertilization (IVF), pre-capacitating sperm prior to *in vitro* fertilization; and
c. the reproductive approach for low ti-fertility status is selected from group consisting of: *in vitro* fertilization (IVF), pre-capacitating sperm prior to *in vitro* fertilization, intracytoplasmic sperm injection (ICSI), pre-capacitating sperm prior to intracytoplasmic sperm injection, gamete intra-fallopian transfer (GIFT), pre-capacitating sperm prior to gamete intra-fallopian transfer, subzonal insemination (SUZI) or pre-capacitating sperm prior to subzonal insemination.

6. The method according to any of claims 2-5, wherein when the reproductive approach corresponds to: (i) pre-capacitating sperm prior to *in vitro* fertilization, (ii) intracytoplasmic sperm injection (ICSI), (iii) gamete intra-fallopian transfer (GIFT), or (iv) subzonal insemination (SUZI), the time period for pre-capacitation prior to insemination corresponds to incubating sperm in media containing one or more stimuli for capacitation, for periods of 24 hours before insemination; 18 hours before insemination; 12 hours before insemination; 6 hours before insemination; 4 hours before insemination; 3 hours before insemination; or 1 hour before insemination.

7. The method according to claim 6, wherein the time period and/or the reproductive approach is also based on one or more of the following: patient demographics, reproductive status of female partner, sperm concentration, total motility, progressive motility, semen volume, semen pH, semen viscosity and/or sperm morphology and combinations thereof.

8. The method of claim 7, wherein the one or more G_{M1} localization patterns include AA G_{M1} localization pattern, APM G_{M1} localization pattern, Lined-Cell G_{M1} localization pattern, INTER G_{M1} localization pattern, PAPM G_{M1} localization pattern, AA/PA G_{M1} localization pattern, ES G_{M1} localization pattern, and DIFF G_{M1} localization pattern.

9. The method of claim 8, where the sperm cells were treated to cryopreservation procedures and stored prior to being treated *in vitro* with capacitation conditions.

## Patentansprüche

1. Verfahren zur Verwendung einer Differenz des Prozentsatzes von to-[AA-G_{M1}-Lokalisierungsmustern plus APM-G_{M1}-Lokalisierungsmustern] und Prozentsatzes von t₁-[AA-G_{M1}-Lokalisierungsmustern plus APM-G_{M1}-Lokalisierungsmustern] als einen Indikator einer Zeitdauer für die Insemination und eines reproduktiven Ansatzes zum Erzielen von Säuger-Fertilisation, das die folgenden Schritte umfasst:
a. Behandeln einer ersten Probe von t₀-*in-vitro*-kapazitierten Spermazellen mit einem Fixativ und einer Fluoreszenzmarkierung;
b. Behandeln einer zweiten Probe von t₁-*in-vitro*-kapazitierten Spermazellen mit einem Fixativ und einer Fluoreszenzmarkierung, wobei die kapazitierten Spermazellen der ersten Probe und der zweiten Probe *in vitro* mit Kapazitationsbedingungen für eine Kapazitationszeitdauer im Bereich zwischen Folgenden behandelt werden: 0,5 Stunden bis 3 Stunden; 3 Stunden bis 12 Stunden; 6 Stunden bis 12 Stunden; 3 Stunden bis 24 Stunden; 12 Stunden bis 24 Stunden; oder 18 Stunden bis 24 Stunden;
c. Erhalten von einem oder mehreren t₀-Fluorenszenzbildern, die ein oder mehrere G_{M1}-Lokalisierungsmuster zeigen, die mit t₀-fluoreszenzmarkierten *in-vitro-*kapazitierten Spermazellen verbunden sind,
d. Erhalten von einem oder mehreren ti-Fluorenszenzbildern, die ein oder mehrere G_{M1}-Lokalisierungsmuster zeigen, die mit t₁-fluoreszenzmarkierten *in-vitro-*kapazitierten Spermazellen verbunden sind, wobei die t₀-Fluorenszenzbilder und t₁-Fluorenszenzbilder zu Zeiten nach *in*-*vitro*-Kapazitation erhalten werden, wobei t₁ größer ist als t₀;
e. Messen einer Anzahl von apikales-Akrosom (AA)-G_{M1}-Lokalisierungsmustern, einer Anzahl von Akrosom-Plasmamembran (APM)-G_{M1}-Lokalisierungsmustern und einer Gesamtanzahl von G_{M1}-Lokalisierungsmustern für die t₀-fluoreszenzmarkierten *in-vitro*-kapazitierten Spermazellen, die in den t₀-Fluorenszenzbildern gezeigt werden, um den Prozentsatz von t₀-[AA-G_{M1}-Lokalisierungsmustern plus APM-G_{M1}-Lokalisierungsmustern] zu bestimmen;
f. Messen einer Anzahl von apikales-Akrosom (AA)-G_{M1}-Lokalisierungsmustern, einer Anzahl von Akrosom-Plasmamembran (APM)-G_{M1}-Lokalisierungsmustern und einer Gesamtanzahl von G_{M1}-Lokalisierungsmustern für die tifluoreszenzmarkierten *in-vitro*-kapazitierten Spermazellen, die in den ti-Fluorenszenzbildern gezeigt werden, um den Prozentsatz von t₁-[AA-G_{M1}-Lokalisierungsmustern plus APM-G_{M1}-Lokalisierungsmustern] zu bestimmen;
g. Vergleichen des Prozentsatzes von t₁-[AA-G_{M1}-Lokalisierungsmustern plus APM-G_{M1}-Lokalisierungsmustern] mit dem Prozentsatz von t₀-[AA-G_{M1}-Lokalisierungsmustern plus APM-G_{M1}-Lokalisierungsmustern], wobei der Vergleich eine *in*-*vivo*-Kapazitationszeit anzeigt, die aus einer späten *in-vivo-*Kapazitationszeit von mehr als 12 Stunden oder einer standardmäßigen *in-vivo-*Kapazitationszeit von 12 Stunden oder weniger ausgewählt ist,
i. wobei eine Differenz des Prozentsatzes von to-[AA-G_{M1}-Lokalisierungsmustern plus APM-G_{M1}-Lokalisierungsmustern] und des Prozentsatzes von t₁-[AA-G_{M1}-Lokalisierungsmustern plus APM-G_{M1}-Lokalisierungsmustern] Folgendem entspricht: mehr als eine Standardabweichung von einem Standard von 35 % zeigt eine späte *in-vivo-*Kapazitationszeit von mehr als 12 Stunden an; oder weniger als eine Standardabweichung von einem Standard von 35 % zeigt eine standardmäßige *in-vivo*-Kapazitationszeit von weniger als 12 Stunden an;
ii. wobei ein Referenzprozentsatz von [AA-G_{M1}-Lokalisierungsmustern plus APM-G_{M1}-Lokalisierungsmustern] Folgendem entspricht: mehr als 35 % zeigt einen Zustand hoher Fertilität an; eine Standardabweichung unter 35 % zeigt einen Zustand mittlerer Fertilität an; und zwei oder mehr Standardabweichungen unter 35 % zeigen einen Zustand niedriger Fertilität an;
iii. weiter wobei eine Differenz des Prozentsatzes von t₀-[AA-G_{M1}-Lokalisierungsmustern plus APM-G_{M1}-Lokalisierungsmustern] und des Prozentsatzes von t₁-[AA-G_{M1}-Lokalisierungsmustern plus APM-G_{M1}-Lokalisierungsmustern] Folgendem entspricht: bei mehr als einer Standardabweichung von einem Standard von 35 % wird ein ti-Fertilitätszustand dann basierend auf einem Vergleich des Prozentsatzes von gemessenem t₁-[AA-G_{M1}-Lokalisierungsmustern plus APM-G_{M1}-Lokalisierungsmustern] mit dem Referenzprozentsatz von [AA-G_{M1}-Lokalisierungsmustern plus APM-G_{M1}-Lokalisierungsmustern] angezeigt; oder bei weniger als einer Standardabweichung von einem Standard von 35 % wird ein ti-Fertilitätszustand dann basierend auf einem Vergleich des Referenzprozentsatzes von [AA-G_{M1}-Lokalisierungsmustern plus APM-G_{M1}-Lokalisierungsmustern] mit dem Prozentsatz von gemessenem t₁-[AA-G_{M1}-Lokalisierungsmustern plus APM-G_{M1}-Lokalisierungsmustern] oder dem Prozentsatz von gemessenem t₀-[AA-G_{M1}-Lokalisierungsmustern plus APM-G_{M1}-Lokalisierungsmustern] angezeigt;
wodurch die Zeitdauer für Insemination und der reproduktive Ansatz für die Verwendung angezeigt wird, um eine Fertilisation basierend auf dem männlichen ti-Fertilitätszustand und der *in-vivo*-Kapazitationszeit zu erzielen.

2. Verfahren nach Anspruch 1, wobei, wenn das Männchen zumindest normale Spermienkonzentration und späte *in-vivo*-Kapazitationszeit aufweist, dann
a. der reproduktive Ansatz für einen Zustand hoher t₁-Fertilität aus der Gruppe ausgewählt ist, die aus Folgenden besteht: Modifizieren der Zeitabstimmung des Geschlechtsverkehrs auf späte *in-vivo*-Kapazitationszeit; Modifizieren der Zeitabstimmung von intrazervikaler Insemination (ICI) auf späte *in-*vivo-Kapazitationszeit; Modifizieren der Zeitabstimmung von intrauteriner Insemination (IUI) auf späte *in-vivo*-Kapazitationszeit; Vorkapazitation von Sperma vor intrazervikaler Insemination oder Vorkapazitation von Sperma vor intrauteriner Insemination;
b. der reproduktive Ansatz für einen Zustand mittlerer t₁-Fertilität aus der Gruppe ausgewählt ist, die aus Folgenden besteht: Modifizieren der Zeitabstimmung von intrazervikaler Insemination (ICI) auf späte *in-vivo-*Kapazitationszeit; Modifizieren der Zeitabstimmung von intrauteriner Insemination (IUI) auf späte *in-vivo*-Kapazitationszeit; Modifizieren der Zeitabstimmung von *in-vitro*-Fertilisation (IVF) auf späte *in-vivo-*Kapazitationszeit; Vorkapazitation von Sperma vor intrazervikaler Insemination; intrauteriner Insemination (IUI); Vorkapazitation von Sperma vor intrauteriner Insemination; oder Vorkapazitation von Sperma vor *in-*vitro-Fertilisation, und
c. der reproduktive Ansatz für einen Zustand niedriger t₁-Fertilität aus der Gruppe ausgewählt ist, die aus Folgenden besteht: Modifizieren der Zeitabstimmung von *in-vitro*-Fertilisation (IVF) auf späte *in-vivo-*Kapazitationszeit; Modifizieren der Zeitabstimmung von intrazytoplasmatischer Spermieninjektion (ICSI) auf späte *in-vivo-*Kapazitationszeit; Modifizieren der Zeitabstimmung von intratubarem Gametentransfer (GIFT) auf späte *in-vivo*-Kapazitationszeit; Modifizieren der Zeitabstimmung von subzonaler Insemination (SUZI) auf späte *in-vivo-*Kapazitationszeit; Vorkapazitation von Sperma vor *in-vitro*-Fertilisation, Vorkapazitation von Sperma vor intrazytoplasmatischer Spermieninjektion, Vorkapazitation von Sperma vor intratubarem Gametentransfer oder Vorkapazitation von Sperma vor subzonaler Insemination.

3. Verfahren nach Anspruch 1, wobei, wenn das Männchen eine kleinere als die normale Spermienkonzentration und späte *in-vivo*-Kapazitationszeit aufweist, dann
a. der reproduktive Ansatz für einen Zustand hoher t₁-Fertilität aus der Gruppe ausgewählt ist, die aus Folgenden besteht: Modifizieren der Zeitabstimmung des Geschlechtsverkehrs auf späte *in-vivo*-Kapazitationszeit; Modifizieren der Zeitabstimmung von intrazervikaler Insemination (ICI) auf späte *in-*vivo-Kapazitationszeit; Modifizieren der Zeitabstimmung von intrauteriner Insemination (IUI) auf späte *in-vivo*-Kapazitationszeit; Vorkapazitation von Sperma vor intrazervikaler Insemination (ICI) oder Vorkapazitation von Sperma vor intrauteriner Insemination (IUI);
b. der reproduktive Ansatz für einen Zustand mittlerer t₁-Fertilität aus der Gruppe ausgewählt ist, die aus Folgenden besteht: Modifizieren der Zeitabstimmung von intrazervikaler Insemination (ICI) auf späte *in-vivo-*Kapazitationszeit; Modifizieren der Zeitabstimmung von intrauteriner Insemination (IUI) auf späte *in-vivo*-Kapazitationszeit; Modifizieren der Zeitabstimmung von *in-vitro*-Fertilisation (IVF) auf späte *in-vivo-*Kapazitationszeit; Vorkapazitation von Sperma vor intrazervikaler Insemination, Vorkapazitation von Sperma vor intrauteriner Insemination; oder Vorkapazitation von Sperma vor *in-vitro*-Fertilisation, und
c. der reproduktive Ansatz für einen Zustand niedriger t₁-Fertilität aus der Gruppe ausgewählt ist, die aus Folgendem besteht: Modifizieren der Zeitabstimmung von *in-vitro*-Fertilisation (IVF) auf späte *in-vivo-*Kapazitationszeit; intrazytoplasmatischer Spermieninjektion (ICSI), Modifizieren der Zeitabstimmung von intrazytoplasmatischer Spermieninjektion (ICSI) auf späte *in-vivo*-Kapazitationszeit; Modifizieren der Zeitabstimmung von intratubarem Gametentransfer (GIFT) auf späte *in-*vivo-Kapazitationszeit; Modifizieren der Zeitabstimmung von subzonaler Insemination (SUZI) auf späte *in-vivo*-Kapazitationszeit, Vorkapazitation von Sperma vor *in-vitro*-Fertilisation, Vorkapazitation von Sperma vor intrazytoplasmatischer Spermieninjektion, Vorkapazitation von Sperma vor intratubarem Gametentransfer oder Vorkapazitation von Sperma vor subzonaler Insemination.

4. Verfahren nach Anspruch 1, wobei, wenn das Männchen zumindest eine normale Spermienkonzentration und standardmäßige *in-vivo*-Kapazitationszeit aufweist, dann
a. der reproduktive Ansatz für einen Zustand hoher t₁-Fertilität aus der Gruppe ausgewählt ist, die aus Folgenden besteht: Geschlechtsverkehr, intrazervikaler Insemination (ICI), Vorkapazitation von Sperma vor intrazervikaler Insemination, intrauteriner Insemination (IUI) oder Vorkapazitation von Sperma vor intrauteriner Insemination;
b. der reproduktive Ansatz für einen Zustand mittlerer t₁-Fertilität aus der Gruppe ausgewählt ist, die aus Folgenden besteht: intrazervikaler Insemination (ICI), Vorkapazitation von Sperma vor intrazervikaler Insemination; intrauteriner Insemination (IUI); Vorkapazitation von Sperma vor intrauteriner Insemination; oder *in-vitro*-Fertilisation (IVF) oder Vorkapazitation von Sperma vor *in-vitro*-Fertilisation, und
c. der reproduktive Ansatz für einen Zustand niedriger t₁-Fertilität aus der Gruppe ausgewählt ist, die aus Folgenden besteht: *in-vitro*-Fertilisation (IVF), Vorkapazitation von Sperma vor *in-vitro*-Fertilisation, intrazytoplasmatischer Spermieninjektion (ICSI), Vorkapazitation von Sperma vor intrazytoplasmatischer Spermieninjektion, intratubarem Gametentransfer (GIFT), Vorkapazitation von Sperma vor intratubarem Gametentransfer, subzonaler Insemination (SUZI) oder Vorkapazitation von Sperma vor subzonaler Insemination.

5. Verfahren nach Anspruch 1, wobei, wenn das Männchen eine geringere als die normale Spermienkonzentration und standardmäßige *in-vivo*-Kapazitationszeit aufweist, dann
a. der reproduktive Ansatz für einen Zustand hoher t₁-Fertilität aus der Gruppe ausgewählt ist, die aus Folgenden besteht: intrazervikaler Insemination (ICI), Vorkapazitation von Sperma vor intrazervikaler Insemination, intrauteriner Insemination (IUI) oder Vorkapazitation von Sperma vor intrauteriner Insemination;
b. der reproduktive Ansatz für einen Zustand mittlerer t₁-Fertilität aus der Gruppe ausgewählt ist, die aus Folgenden besteht: intrazervikaler Insemination (ICI), Vorkapazitation von Sperma vor intrazervikaler Insemination, intrauteriner Insemination (IUI) oder Vorkapazitation von Sperma vor intrauteriner Insemination; *in-vitro*-Fertilisation (IVF), Vorkapazitation von Sperma vor *in-vitro*-Fertilisation, und
c. der reproduktive Ansatz für einen Zustand niedriger t₁-Fertilität aus der Gruppe ausgewählt ist, die aus Folgenden besteht: *in-vitro*-Fertilisation (IVF), Vorkapazitation von Sperma vor *in-vitro*-Fertilisation, intrazytoplasmatischer Spermieninjektion (ICSI), Vorkapazitation von Sperma vor intrazytoplasmatischer Spermieninjektion, intratubarem Gametentransfer (GIFT), Vorkapazitation von Sperma vor intratubarem Gametentransfer, subzonaler Insemination (SUZI) oder Vorkapazitation von Sperma vor subzonaler Insemination.

6. Verfahren nach einem der Ansprüche 2-5, wobei, wenn der reproduktive Ansatz Folgendem entspricht: (i) Vorkapazitation von Sperma vor *in-vitro-*Fertilisation, (ii) intrazytoplasmatischer Spermieninjektion (ICSI), (iii) intratubarem Gametentransfer (GIFT) oder (iv) subzonaler Insemination (SUZI), die Zeitdauer für Vorkapazitation vor der Insemination dem Inkubieren von Sperma in Medien, die einen oder mehrere Stimuli für Kapazitation enthalten, für eine Dauer von Folgenden entspricht: 24 Stunden vor Insemination; 18 Stunden vor Insemination; 12 Stunden vor Insemination; 6 Stunden vor Insemination; 4 Stunden vor Insemination; 3 Stunden vor Insemination; oder 1 Stunde vor Insemination.

7. Verfahren nach Anspruch 6, wobei die Zeitdauer und/oder der reproduktive Ansatz auch auf einem oder mehreren der Folgenden basieren: demographischen Patientendaten, reproduktivem Zustand des weiblichen Partners, Spermienkonzentration, Gesamtmotilität, progressiver Motilität, Samenvolumen, Samen-pH, Samenviskosität und/oder Spermienmorphologie und Kombinationen davon.

8. Verfahren nach Anspruch 7, wobei das eine oder die mehreren G_{M1}-Lokalisierungsmuster Folgende einschließen: AA-G_{M1}-Lokalisierungsmuster, APM-G_{M1}-Lokalisierungsmuster, Lined-Cell-G_{M1}-Lokalisierungsmuster, INTER-G_{M1}-Lokalisierungsmuster, PAPM-G_{M1}-Lokalisierungsmuster, AA/PA-G_{M1}-Lokalisierungsmuster, ES-G_{M1}-Lokalisierungsmuster und DIFF-G_{M1}-Lokalisierungsmuster.

9. Verfahren nach Anspruch 8, wo die Spermienzellen durch Kryokonservierungsverfahren behandelt und vor der Behandlung *in vitro* mit Kapazitationsbedingungen gelagert wurden.

## Revendications

1. Procédé d'utilisation d'une différence dans le pourcentage de [motifs de localisation G_{M1} AA plus motifs de localisation G_{M1} APM]-t₀ et le pourcentage de [motifs de localisation G_{M1} AA plus motifs de localisation G_{M1} APM]-t₁ comme un indicateur de la période de temps pour l'insémination et d'une démarche reproductive afin de réaliser la fécondation mammalienne, comprenant les étapes consistant à :
a. traiter un premier échantillon de spermatozoïdes capacités *in vitro-*t₀ avec un fixateur et une étiquette fluorescente ;
b. traiter un deuxième échantillon de spermatozoïdes capacités *in vitro-*t₁ avec un fixateur et une étiquette fluorescente, dans lequel les spermatozoïdes capacités dans le premier échantillon et le deuxième échantillon sont traités *in vitro* dans des conditions de capacitation pendant une période de temps de capacitation allant d'entre 0,5 heure à 3 heures ; de 3 heures à 12 heures ; de 6 heures à 12 heures ; de 3 heures à 24 heures ; de 12 heures à 24 heures ou de 18 heures à 24 heures ;
c. obtenir une ou plusieurs images de fluorescence-t₀ affichant un ou plusieurs motifs de localisation G_{M1} associés aux spermatozoïdes capacités *in vitro* marqués par flurorescence-t₀ ;
d. obtenir une ou plusieurs images de fluorescence-t₁ affichant un ou plusieurs motifs de localisation G_{M1} associés aux spermatozoïdes capacités *in vitro* marqués par fluorescence-t₁, lesdites images de fluorescence-t₀ et de fluorescence-t₁ étant obtenues à des temps de capacitation post *in vitro,* où t₁ est plus grand que t₀ ;
e. mesurer un nombre de motifs de localisation G_{M1} d'acrosome apical (AA), un nombre de motifs de localisation G_{M1} de membrane plasmatique acrosomique (APM) et un nombre total de motifs de localisation G_{M1} pour les spermatozoïdes capacités *in vitro* marqués par fluorescence-t₀ affichés dans les images de fluorescence-t₀ afin de déterminer le pourcentage de [motifs de localisation G_{M1} AA plus motifs de localisation G_{M1} APM]-t₀ ;
f. mesurer un nombre de motifs de localisation G_{M1} d'acrosome apical (AA), un nombre de motifs de localisation G_{M1} de membrane plasmatique acrosomique (APM) et un nombre total de motifs de localisation G_{M1} pour les spermatozoïdes capacités *in vitro* marqués par fluorescence-t₁ affichés dans les images de fluorescence-t₁ afin de déterminer le pourcentage de [motifs de localisation G_{M1} AA plus motifs de localisation G_{M1} APM]-t₁ ;
g. comparer le pourcentage de [motifs de localisation G_{M1} AA plus motifs de localisation G_{M1} APM]-t₁ au pourcentage de [motifs de localisation G_{M1} AA plus motifs de localisation G_{M1} APM]-t₀, où la comparaison indique un temps de capacitation *in vivo* sélectionné parmi un temps de capacitation *in vivo* tardif plus long que 12 heures ou un temps de capacitation *in vivo* standard de 12 heures ou moins,
i. dans lequel une différence dans le pourcentage de [motifs de localisation G_{M1} AA plus motifs de localisation G_{M1} APM]-t₀ et le pourcentage de [motifs de localisation G_{M1} AA plus motifs de localisation G_{M1} APM]-t₁ correspondant à : plus grande qu'un écart-type d'un standard de 35 % indique un temps de capacitation *in vivo* tardif plus long que 12 heures ; ou moindre qu'un écart-type d'un standard de 35 % indique un temps de capacitation *in vivo* standard de moins de 12 heures ;
ii. dans lequel un pourcentage de référence de [motifs de localisation G_{M1} AA plus motifs de localisation G_{M1} APM] correspondant à : plus grand que 35 % indique un état de fertilité élevée ; un écart-type inférieur à 35 % indique un état de fertilité moyenne ; et deux écarts-types ou plus inférieurs à 35 % indiquent un état de fertilité basse,
iii. dans lequel en outre une différence dans le pourcentage de [motifs de localisation G_{M1} AA plus motifs de localisation G_{M1} APM]-t₀ et le pourcentage de [motifs de localisation G_{M1} AA plus motifs de localisation G_{M1} APM]-t₁ correspondant à : plus grande qu'un écart-type d'un standard de 35 %, un état de fertilité-t₁ est alors indiqué sur la base d'une comparaison du pourcentage des [motifs de localisation G_{M1} AA plus motifs de localisation G_{M1} APM]-t₁ mesurés au pourcentage de référence de [motifs de localisation G_{M1} AA plus motifs de localisation G_{M1} APM] ; ou bien moins d'un écart-type d'un standard de 35 % un état de fertilité-t₁ est alors indiqué sur la base d'une comparaison du pourcentage de référence de [motifs de localisation G_{M1} AA plus motifs de localisation G_{M1} APM] au pourcentage de [motifs de localisation G_{M1} AA plus motifs de localisation G_{M1} APM]-t₁ mesurés ou au pourcentage de [motifs de localisation G_{M1} AA plus motifs de localisation G_{M1} APM]-t₀ mesurés ;
indiquant ainsi la période de temps d'insémination et la démarche reproductive à utiliser afin de réaliser la fécondation sur la base d'un état de fertilité-t₁ du mâle et du temps de capacitation *in vivo.*

2. Procédé selon la revendication 1, dans lequel si le mâle a au moins une concentration de sperme normale et un temps de capacitation *in vivo* tardif alors
a. la démarche reproductive pour un état de fertilité-t₁ élevée est sélectionnée parmi le groupe composé de : modifier le moment choisi de rapport sexuel au temps de capacitation *in vivo* tardif ; modifier le moment d'insémination intracervicale (IIC) au temps de capacitation *in vivo* tardif ; modifier le moment d'insémination intra-utérine (IIU) au temps de capacitation *in vivo* tardif ; pré-capaciter le sperme avant l'insémination intracervicale ou pré-capaciter le sperme avant l'insémination intra-utérine ;
b. la démarche reproductive pour un état de fertilité-t₁ moyenne est sélectionnée parmi le groupe composé de : modifier le moment d'insémination intracervical (IIC) au temps de capacitation *in vivo* tardif ; modifier le moment d'insémination intra-utérine (IIU) au temps de capacitation *in vivo* tardif ; modifier le moment de fécondation *in vitro* (FIV) au temps de capacitation *in vivo* tardif ; pré-capaciter le sperme avant l'insémination intracervicale ; insémination intra-utérine (IIU) ; pré-capaciter le sperme avant l'insémination intra-utérine ; ou pré-capacité le sperme avant la fécondation *in vitro* ; et
c. la démarche reproductive pour un état de fertilité-t₁ basse est sélectionné parmi le groupe composé de : modifier le moment de fécondation *in vitro* (FIV) au temps de capacitation *in vivo* tardif ; modifier le moment d'injection intracytoplasmique de sperme (IICS) au temps de capacitation *in vivo* tardif ; modifier le moment de transfert intratubulaire de gamètes (GIFT) au temps de capacitation *in vivo* tardif ; modifier le moment d'insémination sous-pellucide (SUZI) au temps de capacitation *in vivo* tardif ; pré-capaciter le sperme avant la fécondation *in vitro* ; pré-capaciter le sperme avant l'injection intracytoplasmique de sperme, pré-capaciter le sperme avant le transfert intratubulaire de gamètes ou pré-capaciter le sperme avant l'insémination sous-pellucide.

3. Procédé selon la revendication 1, dans lequel si le mâle a une concentration de sperme inférieure à la normale et un temps de capacitation *in vivo* tardif, alors
a. la démarche reproductive pour un état de fertilité-t₁ élevée est sélectionnée parmi le groupe composé de : modifier le moment choisi de rapport sexuel au temps de capacitation *in vivo* tardif ; modifier le moment d'insémination intracervicale (IIC) au temps de capacitation *in vivo* tardif ; modifier le moment d'insémination intra-utérine (IIU) au temps de capacitation *in vivo* tardif ; pré-capaciter le sperme avant l'insémination intracervicale (IIC) ou pré-capaciter le sperme avant l'insémination intra-utérine (IIU);
b. la démarche reproductive pour un état de fertilité-t₁ moyenne est sélectionné parmi le groupe composé de : modifier le moment d'insémination intracervical (IIC) au temps de capacitation *in vivo* tardif ; modifier le moment d'insémination intra-utérine (IIU) au temps de capacitation *in vivo* tardif ; modifier le moment de fécondation *in vitro* (FIV) au temps de capacitation *in vivo* tardif ; pré-capaciter le sperme avant l'insémination intracervicale ; pré-capaciter le sperme avant l'insémination intra-utérine ; ou pré-capaciter le sperme avant la fécondation *in vitro* ; et
c. la démarche reproductive pour un état de fertilité-t₁ basse est sélectionnée parmi le groupe composé de : modifier le moment de fécondation *in vitro* (FIV) au temps de capacitation *in vivo* tardif ; injection intracytoplasmique de sperme (IICS), modifier le moment d'injection intracytoplasmique de sperme (IICS) au temps de capacitation *in vivo* tardif ; modifier le moment de transfert intratubulaire de gamètes (GIFT) au temps de capacitation *in vivo* tardif ; modifier le moment d'insémination sous-pellucide (SUZI) au temps de capacitation *in vivo* tardif ; pré-capaciter le sperme avant la fécondation *in* vitro, pré-capaciter le sperme avant l'injection intracytoplasmique de sperme, pré-capaciter le sperme avant le transfert intratubulaire de gamètes ou pré-capaciter du sperme avant l'insémination sous-pellucide.

4. Procédé selon la revendication 1, dans lequel si le mâle a une concentration de sperme au moins normale et un temps de capacitation *in vivo* standard, alors
a. la démarche reproductive pour un état de fertilité-t₁ élevée est sélectionnée parmi le groupe composé de : rapport sexuel, insémination intracervicale (IIC), pré-capacitation du sperme avant l'insémination intracervicale, insémination intra-utérine (IIU) ou pré-capacitation du sperme avant l'insémination intra-utérine ;
b. la démarche reproductive pour un état de fertilité-t₁ moyenne est sélectionnée parmi le groupe composé de : insémination intracervicale (IIC), pré-capacitation du sperme avant l'insémination intracervicale ; insémination intra-utérine (IIU) ; pré-capacitation du sperme avant l'insémination intra-utérine ; ou fécondation *in vitro* (FIV) ou pré-capacitation du sperme avant la fécondation *in vitro* ; et
c. la démarche reproductive pour un état de fertilité-t₁ basse est sélectionnée parmi le groupe composé de : fécondation *in vitro* (FIV), pré-capacitation du sperme avant la fécondation *in vitro,* injection intracytoplasmique de sperme (IICS), pré-capacitation du sperme avant l'injection intracytoplasmique de sperme, transfert intratubulaire de gamètes (GIFT), pré-capacitation du sperme avant le transfert intratubulaire de gamètes, insémination sous-pellucide (SUZI), ou pré-capacitation du sperme avant l'insémination sous-pellucide.

5. Procédé selon la revendication 1, dans lequel si le mâle a une concentration de sperme inférieure à la normale et un temps de capacitation *in vivo* standard, alors
a. la démarche reproductive pour un état de fertilité-t₁ élevée est sélectionnée parmi le groupe composé de : insémination intracervicale (IIC), pré-capacitation du sperme avant l'insémination intracervicale, insémination intra-utérine (IIU) ou pré-capacitation du sperme avant l'insémination intra-utérine ;
b. la démarche reproductive pour un état de fertilité-t₁ moyenne est sélectionnée parmi le groupe composé de : insémination intracervicale (IIC), pré-capacitation du sperme avant l'insémination intracervicale, insémination intra-utérine (IIU) ou pré-capacitation du sperme avant l'insémination intra-utérine ; fécondation *in vitro* (FIV), pré-capacitation du sperme avant la fécondation *in vitro* ; et
c. la démarche reproductive pour un état de fertilité-t₁ basse est sélectionnée parmi le groupe composé de : fécondation *in vitro* (FIV), pré-capacitation du sperme avant la fécondation *in vitro,* injection intracytoplasmique de sperme (IICS), pré-capacitation du sperme avant l'injection intracytoplasmique du sperme, transfert intratubulaire de gamètes (GIFT), pré-capacitation du sperme avant le transfert intratubulaire de gamètes, insémination sous-pellucide (SUZI) ou pré-capacitation du sperme avant l'insémination sous-pellucide.

6. Procédé selon l'une quelconque des revendications 2-5, dans lequel lorsque la démarche reproductive correspond à : (i) pré-capaciter le sperme avant la fécondation *in vitro,* (ii) injection intracytoplasmique du sperme (IICS), (iii) transfert intratubulaire de gamètes (GIFT), ou (iv) insémination sous-pellucide (SUZI), la période de temps de pré-capacitation avant l'insémination correspond à incuber le sperme dans des milieux contenant un ou plusieurs stimulants de capacitation, pendant des périodes de 24 heures avant l'insémination ; 18 heures avant l'insémination ; 12 heures avant l'insémination ; 6 heures avant l'insémination ; 4 heures avant l'insémination ; 3 heures avant l'insémination ou 1 heure avant l'insémination.

7. Procédé selon la revendication 6, dans lequel la période de temps et/ou la démarche reproductive sont aussi basées sur l'un ou plusieurs des éléments suivants : démographie du patient, état reproductif de la partenaire femelle, concentration de sperme, motilité totale, motilité progressive, volume de semence, pH de la semence, viscosité de la semence et/ou morphologie du sperme et des combinaisons de ceux-ci.

8. Procédé selon la revendication 7, dans lequel l'un ou plusieurs motifs de localisation G_{M1} comprennent un motif de localisation G_{M1} AA, un motif de localisation G_{M1} Lined-Cell, un motif de localisation G_{M1} INTER, un motif de localisation G_{M1} PAPM, un motif de localisation G_{M1} AA/PA, un motif de localisation G_{M1} ES et un motif de localisation G_{M1} DIFF.

9. Procédé selon la revendication 8, dans lequel les spermatozoïdes sont traités par des procédures de cryoconservation et stockés avant d'être traités *in vitro* dans des conditions de capacitation.
